(19) Europäisches Patentamt / European Patent Office / Office européen des brevets

(11) **EP 2 383 335 B1**

## (12) EUROPEAN PATENT SPECIFICATION

(45) Date of publication and mention of the grant of the patent:
**16.08.2017  Bulletin 2017/33**

(21) Application number: **10731300.9**

(22) Date of filing: **15.01.2010**

(51) Int Cl.:
*C12N 15/09* (2006.01)     *C12Q 1/68* (2006.01)

(86) International application number:
**PCT/JP2010/050426**

(87) International publication number:
**WO 2010/082631 (22.07.2010 Gazette 2010/29)**

(54) **METHOD FOR PRODUCING NUCLEIC ACID SAMPLE AND METHOD FOR PRODUCING NUCLEIC ACID AMPLIFICATION PRODUCT USING THE SAME**

VERFAHREN ZUR HERSTELLUNG EINER NUKLEINSÄUREPROBE UND VERFAHREN ZUR HERSTELLUNG EINES NUKLEINSÄUREAMPLIFIKATIONSPRODUKTES MIT DER NUKLEINSÄUREPROBE

PROCÉDÉ DE PRODUCTION D'ÉCHANTILLON D'ACIDE NUCLEIQUE, ET PROCÉDÉ DE PRODUCTION DE PRODUIT D'AMPLIFICATION D'ACIDE NUCLEIQUE UTILISANT L'ÉCHANTILLON D'ACIDE NUCLEIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.01.2009  JP 2009008091**

(43) Date of publication of application:
**02.11.2011  Bulletin 2011/44**

(73) Proprietor: ARKRAY, Inc.
**Minami-ku**
**Kyoto-shi**
**Kyoto 601-8045 (JP)**

(72) Inventor: **KOZUKA, Masahiro**
**Kyoto 601-8045 (JP)**

(74) Representative: **Jones, Elizabeth Louise**
**Dehns**
**St Bride's House**
**10 Salisbury Square**
**London**
**EC4Y 8JD (GB)**

(56) References cited:
| | |
|---|---|
| EP-A1- 1 416 047 | EP-A1- 1 719 816 |
| WO-A1-02/097084 | WO-A1-2004/094634 |
| JP-A- 11 146 783 | JP-A- 2003 204 799 |
| JP-A- 2004 073 193 | JP-T- 2006 517 298 |
| US-A1- 2005 037 351 | |

SEQUENCE LISTING

<110> ARKRAY, INC.

<120> Method of Producing Nucleic Acid Sample, and Method of Producing Amplified Nucleic Acid Used the Same

<130> TF09046-01

<150> JP09/008091
<151> 2009-01-16

<160> 2

<170> PatentIn version 3.1

<210> 1
<211> 20
<212> DNA
<213> Artificial

<220>
<223> primer for beta-actin

<400> 1
tgaaccccaa ggccaaccgc      20

<210> 2
<211> 20
<212> DNA
<213> Artificial

<220>
<223> primer for beta-actin

<400> 2
ttgtgctggg tgccagggca        20

**Description**

Technical Field

The present invention relates to a method for producing a nucleic acid sample by recovering nucleic acid from a cell sample containing cells and a method for producing a nucleic acid amplification product using the same.

Background Art

In medical fields, as a method for diagnosing, at a genetic level, an infection, a genetic disease, or the like, a method for analyzing a specific mutation or a single nucleotide polymorphism (SNP) by performing nucleic acid amplification with nucleic acid such as DNA or the like collected from a patient being used as a template has been widely used. Nucleic acid is generally recovered from the whole blood of a patient. The whole blood contains various components such as erythrocytes and blood plasma, and some of these components affect later amplification reactions if they remain in large amounts. Further, nucleic acid is generally contained in leukocytes, and there is a possibility that remains of the leukocytes after releasing the nucleic acid therefrom also affect later amplification reactions. Therefore, when recovering nucleic acid serving as a template from a blood sample of a patient, it is required to release nucleic acid from leukocytes and to remove undesired components to some extent.

Recently, there has been a demand for a reduction in the sizes of gene analyzers and tools such as chips used for the gene analyzers. In response to this, it has also been required to recover nucleic acid serving as a template efficiently from a small amount of whole blood. Since there has been demands for removal of undesired components and highly-efficient recovery of nucleic acid from a small amount of a biological sample, the following methods have been disclosed.

The first method is a method for binding a nucleic acid to silica particles in the presence of high levels of a chaotropic salt (Patent Document 1). According to this method, since the nucleic acid is bound to silica particles, it can be recovered by separating a complex of the nucleic acid and the silica particles from a liquid fraction. However, since the chaotropic salt is a denaturing agent having corrosivity, influences of the chaotropic salt on human bodies and environments have been perceived as a problem. Further, although the chaotropic salt is necessary for binding the nucleic acid and the silica particles, it inhibits reactions of various enzymes such as DNA polymerase and restriction enzymes. Therefore, washing with nucleic acid-insoluble organic solvents such as ethanol is required. However, since organic solvents such as ethanol also inhibit various enzymatic reactions, it needs to be removed completely by drying, for example, and there is a problem that the operation becomes complicated. Further, the organic solvents are difficult to handle because they are normally volatile. Moreover, according to this method, carriers for recovering the nucleic acid are limited to particles of silica and their derivatives, and reagents are limited to special reagents containing a chaotropic salt. Therefore, other carriers or reagents cannot be used in place of these, and there are problems of cost and time for preparation.

The second method is a method for binding DNA in a biological sample to a support to recover the nucleic acid by bringing the biological sample into contact with a bead-shaped support in the presence of a surfactant (Patent Document 2). According to this method, DNA can be recovered as a DNA-bead complex in the form of a gel. However, in this method, a large amount of cell suspension is required to be treated with a surfactant for releasing a sufficient amount of DNA from the biological sample. As a result, the amount of the sample may be increased and the sample may not be brought into contact with the beads efficiently. Therefore, it requires pretreatment for enriching cells by centrifugal filtration or the like or enriching desired cells after removing undesired cells and substances, and this requires complicated operations. Further, there is a problem that this complex is easily broken by an operation such as pipetting or vortex mixing, and the yield of DNA is significantly decreased. Therefore, it is required to separate DNA from a liquid phase by a gentle operation or it is required to separate DNA from a liquid phase, using magnetic beads, by recovering the magnetic beads to which the DNA is bound with a magnet.

The third method is a method for causing nonwoven fabric to adsorb the nucleic acid in a cell extract by bringing the cell extract into contact with the nonwoven fabric (Patent Document 3). However, according to this method, the cell extract should be of a high salt concentration for causing the nonwoven fabric to adsorb the nucleic acid. Therefore, when the obtained nucleic acid is used for nucleic acid amplification, for avoiding influences due to salt, for example, the salt should be removed beforehand, and this requires complicated operations. Further, since the nonwoven fabric adsorbs the nucleic acid very strongly, elution of DNA from the nonwoven fabric is difficult. Therefore, the elution requires treatment under extreme environmental conditions. For example, the elution requires long hours of heat treatment under strong alkaline conditions of such as pH12, treatment with reactive oxygen, and the like. Such a method not only requires complicated operations but also has an extremely high possibility of causing damage to the nucleic acid by the elution.

Prior Art Document

Patent Document

Patent Document 1: JP 2680462 B
Patent Document 2: JP 3787354 B
Patent Document 3: WO 03/006650

Disclosure of the Invention

Problem to be Solved by the Invention

Hence, the present invention is intended to provide a simple method for producing a nucleic acid sample that does not need to use high-risk chaotropic salts in large amounts, does not need to use a limited special carrier, and offers a superior level of safety, for example; and a method for producing a nucleic acid amplification product using the same.

Means for Solving Problem

The nucleic acid sample production method of the present invention is a method for producing a nucleic acid sample by recovering nucleic acid from cells, comprising the steps of:

(A) with respect to a cell sample containing cells, releasing nucleic acid complexes from the cells;
(B) contacting a treatment liquid containing the cell sample from step (A) with a carrier;
(C) separating the carrier from the treatment liquid;
(D) applying heat treatment to the carrier; and
(E) adding a dispersion medium to the carrier before or after step (D).

The nucleic acid amplification product production method of the present invention is a method for producing a nucleic acid amplification product having a target sequence by a nucleic acid amplification method, comprising the steps of:

(a) recovering a nucleic acid sample from a cell sample containing cells by the method for producing a nucleic acid sample of the present invention; and
(b) producing, using nucleic acid in the nucleic acid sample recovered in step (a) as a template, a nucleic acid amplification product having a target sequence in the template nucleic acid by a nucleic acid amplification method.

Effects of the Invention

According to the present invention, nucleic acid can be recovered efficiently by releasing nucleic acid complexes from cells. Therefore, for example, besides genomic DNA and RNA, mitochondrial DNA also can be recovered. Further, since release of the nucleic acid complexes from the cells can be performed without using a special reagent, for example, the present invention is superior in safety and operability. In this manner, since the present invention is superior in the recovery rate of nucleic acid as well as in safety and operability, for example, a sufficient amount of nucleic acid serving as a template required for nucleic acid amplification or the like can be recovered from a small amount of cells and a small amount of cell sample. Further, since the recovery of nucleic acid from a small amount of cells or a small amount of cell sample can be performed efficiently by a simple operation, for example, it may be said that the present invention is useful for nucleic acid amplification and nucleic acid recovery using a microchip, micro-TAS, or the like, which have received attention recently.

Brief Description of Drawings

[FIG. 1] FIG. 1 is a cross-sectional view showing an example of a nucleic acid recovery pipette chip of the present invention.

[FIG. 2] FIGs. 2A to 2D are schematic views showing the outline of a nucleic acid recovery method using a nucleic acid recovery pipette chip of the present invention.

[FIG. 3] FIGs. 3A to 3D are schematic views showing an example of a nucleic acid recovery biochip of the present invention.

[FIG. 4] FIGs. 4A to 4D are schematic views showing an example of a nucleic acid amplification biochip of the present invention.

[FIG. 5] FIGs. 5A to 5C are schematic views showing an example of a jig used in Example 1 of the present invention; and FIG. 5A is a top view, FIG. 5B is a cross-sectional view, and FIG. 5C is a schematic view indicating the direction of a liquid passing through a carrier.

[FIG. 6] FIGs. 6A to 6D are schematic views showing an example of a jig used in Example 2 of the present invention; and FIGs. 6A and 6B are perspective views, FIG. 6C is a top view, and FIG. 6D is a cross-sectional view.

[FIG. 7] FIG. 7 is a micrograph showing Giemsa staining of a nucleic acid complex in the reference example.

Description of Embodiments

<Nucleic Acid Sample Production Method>

As described above, the nucleic acid sample production method of the present invention is a method for producing a nucleic acid sample by recovering nucleic acid from cells, comprising the steps of

(A) with respect to a cell sample containing cells, releasing nucleic acid complexes from the cells;
(B) bringing a treatment liquid containing the cell sample from step (A) into contact with a carrier;
(C) separating the carrier from the treatment liquid;
(D) applying heat treatment to the carrier; and
(E) adding a dispersion medium to the carrier before or after step (D).

The nucleic acid sample production method of the present invention also may be referred to as a nucleic acid sample preparation method, a nucleic acid sample recovery method, or a nucleic acid recovery method from cells.

In the production method of the present invention, it is presumed that recovery of nucleic acid is performed by the following mechanism. However, the present invention is not limited thereto. In conventional nucleic acid recovery, particularly in genomic DNA recovery, normally, breaking of nuclei in cells (for example, leukocytes), release of chromosomes packaged in the nuclei, linearization of the nuclear genome by removal of undesired proteins, fibers, and the like are firstly performed as treatment of a sample such as blood or the like, and then, the nuclear genome is recovered by causing the nuclear genome to be held in various carriers and separating the carriers from a liquid fraction, for example. In contrast, in the present invention, although the chromosome-containing nuclear genome is released from the nuclei of cells, linearization of the nuclear genome is not performed, and the nuclear genome is maintained as a particle-shaped nucleic acid complex that is highly conformational, in which the nuclear genome is entwined. This nucleic acid complex normally has a diameter of from 50 $\mu$m to 200 $\mu$m. This nucleic acid is larger than a nucleus having a diameter of 10 $\mu$m or shorter, has larger steric hindrance than a linear nuclear genome, and is highly conformational. Therefore, it can easily be held in a carrier, for example. Accordingly, cell-derived nuclear genome can efficiently be captured by a carrier. Further, by capturing the nuclear genome in a state of a nucleic acid complex by the carrier, mitochondrial genome also can be recovered. The mitochondrial genome is cyclic DNA in which about 17, 000 nucleotides are connected, and is very sensitive. Therefore, it has been very difficult to recover the mitochondrial genome. According to the present invention, since a nucleic acid complex captured by the carrier has a structure in which the nucleic acid is entwined, it is presumed that the mitochondrial genome is further captured in this structure. Further, since such a nucleic acid complex is very superior in stability as compared to a linear nuclear genome, for example, the complex is hardly broken by operations such as pipetting and vortex mixing. Therefore, the decrease in the recovery rate due to a recovery operation can be prevented. Further, by simply applying heat treatment to nucleic acid complexes captured by the carrier, various nucleic acids such as genomic DNA, mitochondrial DNA, plasmid DNA, and RNA contained in the nucleic acid complexes can be recovered in a dispersion medium.

The method for bringing the treatment liquid from step (A) into contact with the carrier in step (B) and the method for separating the carrier from the treatment liquid in step (C) are not particularly limited, and can be decided suitably depending on the shape, the arrangement, or the like of the carrier, for example, as will be described below. In step (C), the separation of the carrier from the treatment liquid can also be referred to as separation of the carrier from a liquid fraction or removal of the treatment liquid or the liquid fraction. The treatment liquid (liquid fraction) is not necessarily removed from the carrier completely, and the treatment liquid may remain in the carrier, for example.

As described above, addition of a dispersion medium in step (E) may be performed before or after step (D) in which the carrier is applied with heat treatment. In the case where step (E) is performed after step (D), the production method of the present invention is conducted, for example, by performing steps (A) to (C) in this order, followed by step (D) of the heat treatment, and then followed by step (E) of the addition of the dispersion medium. In the case where step (E) is performed before step (D), the production method of the present invention is conducted, for example, by performing steps (A) to (C) in this order, followed by step (E) of the addition of the dispersion medium, and then followed by step (D) of the heat treatment.

(First Embodiment)

In the production method of a nucleic acid sample of the present invention, means for releasing nucleic acid complexes in step (A) are not particularly limited. An example of the production method of a nucleic acid sample of the present invention includes the first production method in which step (A) is a step (A1) of mixing a cell sample with a treatment reagent that contains protease and a surfactant, for example. In the first production method, for example, by mixing the cell sample with the treatment reagent in step (A) in this manner, a treatment liquid in which nucleic acid complexes are released from the cells can be obtained.

The first production method of the present invention will be described hereinafter. However, the present invention is not limited thereto. In the present invention, hereinafter, a reagent, containing protease and a surfactant, prior to mixing with the cell sample is referred to as a "treatment reagent", and a mixture of the treatment reagent and the cell sample is referred to as a "treatment liquid" or a "mixed treatment liquid". In the present invention, mixing of the cell sample and the treatment reagent may be performed, for example, by adding the treatment reagent to the cell sample or adding the cell sample to the treatment reagent. The treatment reagent used in the first production method of the present invention is also referred to as a "first treatment reagent".

Preferably, in the treatment liquid in which the treatment reagent and the cell sample are mixed, the concentration of the protease is, for example, 0.5 mU/$\mu$L or more and the concentration of the surfactant is, for example, 0.1 vol% or more. By treating the cell sample under the foregoing conditions, the nucleic acid complexes can be released sufficiently. The concentration of the protease and the concentration of the surfactant are the concentrations in the treatment liquid containing the treatment reagent and the cell sample and are the concentrations in the case of assuming that the amount of the cell in the treatment liquid is $1 \times 10^2$ to $1 \times 10^9$, for example. Further, the concentration of the protease and the concentration of the surfactant are the concentrations in the treatment liquid containing the treatment reagent and the cell sample and may be the concentrations in the case of assuming that the amount of the cell sample in the treatment liquid is 50 to 100 $\mu$L and preferably 50 $\mu$L.

In the treatment liquid, the lower limit of the concentration of the protease is, for example, 0.5 mU/$\mu$L or more, preferably 1 mU/$\mu$L or more, and more preferably 2 mU/$\mu$L or more. In the treatment liquid, the upper limit of the concentration of the protease is, for example, 1 U/$\mu$L or less, and preferably 500 mU/$\mu$L or less, although it is not particularly limited. The concentration of the protease is, for example, in the range from 0.5 mU/$\mu$L to 1000 mU/$\mu$L, preferably in the range from 1 mU/$\mu$L to 1000 mU/$\mu$L, more preferably in the range from 2 mU/$\mu$L to 500 mU/$\mu$L, and particularly preferably in the range from 5 mU/$\mu$L to 15 mU/$\mu$L. With respect to the unit of protease activity (U: unit), generally, 1 U denotes the enzyme level that allows peptide to be produced corresponding to 1 $\mu$mol of tyrosine in 1 minute at 37°C with hemoglobin being used as a substrate.

In the treatment liquid, the amount of the protease relative to $1 \times 10^2$ to $1 \times 10^9$ cells is not particularly limited. The lower limit thereof is, for example, 0.02 mU or more and preferably 0.08 mU or more, and the upper limit thereof is, for example, 100 U or less and preferably 50 U or less. Further, in the treatment liquid, the amount of the protease relative to 50 $\mu$L of the cell sample is not particularly limited. The lower limit thereof is, for example, 0.02 mU or more and preferably 0.08 mU or more, and the upper limit thereof is, for example, 100 U or less and preferably 50 U or less.

In the treatment liquid, the lower limit of the concentration of the surfactant is, for example, 0.1 vol% or more and preferably 0.2 vol% or more, although it is not particularly limited. Further, in the treatment liquid, the upper limit of the surfactant is, for example, 20 vol% or less, preferably 10 vol% or less, more preferably 5 vol% or less, and yet more preferably 2 vol% or less, although it is not particularly limited. The concentration of the surfactant is, for example, in the range from 0.1 vol% to 20 vol%, preferably in the range from 0.1 vol% to 5 vol%, more preferably in the range from 0.1 vol% to 2 vol%, and particularly preferably in the range from 0.2 vol% to 0.8 vol%.

In the treatment liquid, the amount of the surfactant relative to $1 \times 10^2$ to $1 \times 10^9$ cells is not particularly limited. The lower limit thereof is, for example, 1 femtomole ($1 \times 10^{-15}$ mole) or more and preferably 2 femtomoles or more, and the upper limit thereof is, for example, 200 femtomoles or less and preferably 100 femtomoles or less. Further, in the treatment liquid, the amount of the surfactant relative to 50 $\mu$L of the cell sample is not particularly limited. The lower limit thereof is, for example, 1 femtomole or more and preferably 2 femtomoles or more, and the upper limit thereof is, for example, 200 femtomoles or less and preferably 100 femtomoles or less.

In the case where the cells are eukaryotic cells (nucleated cells), the amounts of the protease and the surfactant preferably are the amounts relative to $1 \times 10^2$ to $1 \times 10^8$ cells and more preferably are the amounts relative to $1 \times 10^3$ to $1 \times 10^7$ cells. Particularly, in the case where whole blood-derived cells (for example, leukocytes) from the eukaryotic cells are used as the cells, the amounts of the protease and the surfactant preferably are the amounts relative to $5\times10^3$ to $1\times10^7$ cells and more preferably are the amounts relative to $2.5\times10^4$ to $1\times10^7$ cells. Particularly, in the case where saliva-derived cells from the eukaryotic cells are used as the cells, the amounts of the protease and the surfactant preferably are the amounts relative to $1\times10^2$ to $1x10^7$ cells and more preferably are the amounts relative to $1\times10^3$ to $1\times10^6$ cells. In the case where the cells are prokaryotic cells, the amounts of the protease and the surfactant preferably are the amounts relative to $1\times10^3$ to $1\times10^9$ cells and more preferably are the amounts relative to $1\times10^3$ to $1\times10^8$ cells, and, for example, the same applies to Escherichia coli and the like.

The concentration of the protease and the concentration of the surfactant in the treatment reagent are not particularly limited. Preferably, the concentrations are set such that the aforementioned concentrations can be obtained in the treatment liquid when the treatment reagent and the cell sample are mixed.

The concentration of the protease in the treatment reagent is not particularly limited. The lower limit thereof is, for example, 1 mU/$\mu$L or more, preferably 2 mU/$\mu$L or more, and more preferably 4 mU/$\mu$L or more. The upper limit thereof is, for example, 2 U/$\mu$L or less and preferably 1 U/$\mu$L or less. The concentration is, for example, in the range from 1 mU/$\mu$L to 2000 mU/$\mu$L, preferably in the range from 2 mU/$\mu$L to 2000 mU/$\mu$L, more preferably in the range from 4 mU/$\mu$L to 1000 mU/$\mu$L, and particularly preferably in the range from 10 mU/$\mu$L to 30 mU/$\mu$L. The concentration of the surfactant in the treatment reagent is not particularly limited. The lower limit thereof is, for example, preferably 0.2 vol% or more. The upper limit thereof is, for example, 40 vol% or less, preferably 20 vol% or less, more preferably 10 vol% or less, and particularly preferably 4 vol% or less. The concentration is, for example, in the range from 0.2 vol% to 40 vol%, preferably in the range from 0.2 vol% to 10 vol%, more preferably in the range from 0.2 vol% to 4 vol%, and particularly preferably in the range from 0.4 vol% to 1.6 vol%.

The ratio between the cell sample (S) and the treatment reagent (T) (volume ratio S : T) is, for example, 1 : 0.5 to 1 : 20, preferably 1 : 0.5 to 1 : 10, and more preferably 1 : 0.5 to 1 : 5, although it is not particularly limited.

Examples of the protease include proteinase K, chymotrypsin, pepsin, cathepsin D, and papain. One of these proteases may be used alone or two or more of them may be used in combination.

The surfactant is not particularly limited and is preferably a nonionic surfactant, for example. Examples of the nonionic surfactant include polyoxyethylene-p-isooctylphenol, polyoxyethylene sorbitan monolaurate, polyoxyethylene-nonylphenyl ether, and nonylphenol polythioethoxylate. Examples of these surfactants include the Triton® series such as Triton® X-100 and Triton® X-114; the Nonidet® series such as Nonidet® P40; and the Tween® series such as Tween® 20 and Tween® 80. One of these surfactants may be used alone or two or more of them may be used in combination.

The form of the treatment reagent is not particularly limited, and may be solid (a drying substance) or liquid. In the latter case, preferably, the protease and the surfactant are added to a solvent, for example. Examples of the solvent include, but are not limited to, aqueous solvents such as water, various buffer solutions, and buffered saline; organic solvents; and mixtures thereof. Examples of the buffer solutions include Tris-HCl, BES, PIPES, SSC, TAE, TBE, TG, MES, Bis-Tris, ADA, ACES, MOPSO, MOPS, TES, HEPES, DIPSO, TAPSO, POPSO, HEPPSO, EPPS, Tricine, Bicine, TAPS, CHES, CAPSO, CAPS, PBS, boric acid, cacodylic acid, citric acid, glycine, glycylglycine, imidazole, lithium citrate, potassium phosphate, sodium citrate, sodium phosphate, and trithylammonium acetate. The concentration of the buffer solution in the treatment reagent is, for example, 0.1 mmol/L to 100 mmol/L, more preferably 0.1 mmol/L to 50 mmol/L, and particularly preferably 0.1 mmol/L to 20 mmol/L, although it is not particularly limited. The pH of the treatment reagent is, for example, 7.8 to 9.5 and preferably 8.5 to 8.9.

The treatment reagent may further contain, for example, a protein denaturing agent. Examples of the protein denaturing agent include urea, $\beta$-mercaptoethanol, sodium lauryl sulfate, dithiothreitol, and guanidinium hydrochloride. The concentration of the protein denaturing agent in the treatment reagent is, for example, 0 mol/mL to 8 mol/mL, preferably 1 mol/mL to 5 mol/mL, and more preferably 1 mol/mL to 3 mol/mL, although it is not particularly limited. One of the protein denaturing agents may be used alone or two or more of them may be used in combination.

The treatment reagent may further contain, for example, a chelating agent. Examples of the chelating agent include EDTA, EGTA, NTA, DTPA, GLDA, HEDTA, GEDTA, TTHA, HIDA, and DHEG. The concentration of the chelating agent in the treatment reagent is, for example, 0 mmol/mL to 10 mmol/mL, preferably 0 mmol/mL to 1 mmol/mL, and more preferably 0 mmol/mL to 0.1 mmol/mL, although it is not particularly limited.

The cell is applicable as long as it contains nucleic acid, for example, and examples thereof include eukaryotic cells and prokaryotic cells. Examples of the eukaryotic cells include blood cells such as leukocytes; cells in a mouth; somatic cells such as cells in a nail and cells in a hair; germ cells; tumor cells. Further, cultured cells of these also can be employed. Examples of the cell sample containing the eukaryotic cells as the cells include, but not limited to, blood, saliva, oral mucosa, nails, and hairs. Further, the cell sample may be cultures of the eukaryotic cells. An example of the prokaryotic cell includes, but is not limited to, Escherichia coli. Further, cultured cells of the Escherichia coli also can be employed. The cell sample containing the prokaryotic cells as the cells is not particularly limited, and may be cultures of the prokaryotic cells, for example. Examples of the cultures include culture solutions of cells and recovered cultured cells. The cell sample may be an undiluted cell sample or a diluted cell sample, for example. The present invention is preferably applied to the recovery of nucleic acid from the undiluted cell sample. In the case where the cell sample includes cells in a mouth or cultured cells recovered after culture, for example, saline or a buffer solution in which the cells are preliminarily suspended, as the cell sample, may be mixed with the treatment reagent or the cells may directly be mixed with the treatment reagent as the cell sample. Further, the cell sample may be a cell sample right after collection or a cell sample that has been allowed to stand at room temperature, refrigerated, or freeze-thawed.

Examples of the blood include whole blood, hemolyzed whole blood, blood cell fractions of whole blood, and dispersion liquids of the blood cell fractions. The blood may be undiluted blood or diluted blood. The present invention is preferably applied to the recovery of nucleic acids from undiluted blood, for example. The blood may be blood right after collection or blood that has been allowed to stand at room temperature, refrigerated, or freeze-thawed.

The nucleic acid recovered by the present invention is at least one of DNA and RNA, for example. Besides genomic DNA, examples of the DNA include plasmid DNA and mitochondrial DNA.

In step (B), a treatment liquid containing the cell sample from step (A), e.g., a treatment liquid in which the treatment reagent and the cell sample are mixed is brought into contact with a carrier. The carrier used in step (B) is not particularly limited. For example, the carrier is applicable as long as it can capture the nucleic acid complexes released from the cells in step (A) by treatment with the treatment reagent. The average diameter of the nucleic acid complexes is, for example, 50 $\mu$m to 200$\mu$m. Therefore, the carrier preferably has a void through which the nucleic acid complexes can not pass easily but the liquid fraction can be passed. Thereby, for example, the liquid fraction can be removed while holding the nucleic acid complexes. Further, since the nucleic acid complex is highly conformational as described above, for example, fiber or the like is more likely to adsorb the nucleic acid complex, i.e., the nucleic acid complex is more likely to be tangled with the fiber or the like. Therefore, the size of the void of the carrier is not particularly limited. For example, the nucleic acid complex may be held by causing the carrier itself (e.g., fibers such as woven fabric and nonwoven fabric) to adsorb the nucleic acid complex. Since the nucleic acid complex is highly conformational, it is presumed that other nucleic acid complexes may further be held by the nucleic acid complex adsorbed to the carrier.

Examples of the carrier include nonwoven fabric; woven fabric such as mesh type woven fabric; and porous bodies such as sponges. The shape thereof also is not particularly limited, and examples thereof include a sheet, a film, a block, and a bead. For example, the bead-shaped carrier may be formed of nonwoven fabric, woven fabric, or a porous body, or may be formed of a non-porous body.

Properties of the aforementioned carriers will be described below. However, the present invention is not limited thereto.

< Nonwoven fabric >

The average fiber diameter is, for example, 20 nm to 100 $\mu$m and preferably about 2 $\mu$m.
The basis weight (area:weight) is, for example, 20 g/m$^3$ to 150 g/m$^3$ and preferably about 20 g/m$^3$.

< Woven fabric >

The mesh size is, for example, 10 $\mu$m to 1000 $\mu$m and preferably 100 $\mu$m to 250 $\mu$m,
[0042] The thread diameter (wire diameter) is, for example, 40 $\mu$m to 500 $\mu$m and preferably 47 $\mu$m to 152 $\mu$m.
The mesh opening size is, for example, 50 $\mu$m to 300 $\mu$m and preferably 50 $\mu$m to 105 $\mu$m.

< Porous body >

The average pore size is, for example, 10 $\mu$m to 1000 $\mu$m and preferably 30 $\mu$m to 1000 $\mu$m.
The average density is, for example, 15 kg/m$^3$ to 85 kg/m$^3$ and preferably 30 kg/m$^3$ to 80 kg/m$^3$.
The pore shape is, for example, a closed cell foam or open cell foam.

< Bead >

The shape includes spheres such as a true sphere and an oval sphere.
The diameter (particle size) is, for example, 30 nm to 100 $\mu$m and preferably about 5 $\mu$m.
The surface area ratio is, for example, 0.5 m$^2$/g to 200 m$^2$/g and preferably 1.1 m$^2$/g.
The filling fraction is, for example, 0.1% to 10% and preferably 0.1% to 5%.
For example, a polymer carrier can be employed as the carrier.

Examples of the polymer include, but not limited to, polypropylene, polyethylene, polyester, polyamide (for example, product name: nyron), polyurethane, polyether, polystyrene, polyvinyl chloride, and polytetrafluoroethylene such as Teflon®. Among them, polytetrafluoroethylene, polypropylene, polyamide, polyurethane, and polyester are preferable, and polypropylene, polyurethane, and polyester are more preferable.

The polymer carrier may be a carrier, the whole body thereof is formed of polymer; or the polymer carrier may be a carrier, only a part thereof to be brought into contact with blood, e.g. , only the exposed surface is coated with polymer.

The bead-shaped polymer carrier may be formed of a porous body or a non-porous body, as described above. As the bead-shaped polymer carrier, for example, a magnetic bead is also preferable because recovery by means of a magnet can be performed. Examples of the polymer comprising the magnetic bead include, but not limited to, polystyrene, polyethylenimine (PEI), polymethyl methacrylate (PMMA), polylactic acid (PLA), chitosan, and copolymers of these polymers and a maleic acid. Further, the magnetic bead may be applied with a surface treatment, for example, with PEG-COOH, alkyl-OH, $SO_3H$, or $SiO_2$. Moreover, with respect to the magnetic bead, for example, the surface of the bead composed of a magnetic body such as metal may be coated with the aforementioned polymer. Examples of the metal include magnetite, maghemite, iron, and nickel.

Besides the aforementioned carriers, for example, a metal carrier can be employed as the carrier. Examples of the metal include stainless steel, titanium, and aluminum. One of these metals may be used alone or two or more of them may be used in combination.

The carrier may be a monolayer or a laminate of two or more layers, for example. The laminate may be composed of one kind of component, or may be composed of two or more kinds of components. Further, the laminate may be composed of components of different materials.

The size of the carrier to be used is not particularly limited and can be decided suitably depending on the amount of the cell sample in step (A), the amount of the treatment liquid in which the cell sample and the treatment reagent are mixed, and the shape of the carrier, for example. Specific examples will be described below.

The method for bringing the treatment liquid into contact with the carrier in step (B) and the method for separating the carrier from the treatment liquid in step (C) are not particularly limited, and can be decided suitably depending on the shape, the arrangement, or the like of the carrier, for example, as will be described below. As described above, the treatment liquid is not necessarily separated from the carrier completely.

The first production method of a nucleic acid sample of the present invention will be described with reference to a method for recovering a nucleic acid from whole blood as an example. However, the present invention is not limited thereto.

Blood Treatment Step

First, the treatment reagent is added to whole blood. Thereby, nucleic acid complexes are released from leukocytes of the whole blood. The nucleic acid complex contains, for example, mitochondrial DNA besides genomic DNA and RNA.

Conditions for treatment with the treatment reagent are as follows: the treatment time is, for example, 1 second to 600 seconds, preferably 5 seconds to 600 seconds, and more preferably 5 seconds to 300 seconds; and the treatment temperature is, for example, 4°C to 60°C, preferably 10°C to 60°C, and more preferably 15°C to 40°C. However, the conditions are not particularly limited thereto.

Contact Stew with Carrier

Subsequently, the treatment liquid in which the whole blood and the treatment reagent are mixed is brought into contact with the carrier. Thereby, nucleic acid complexes released from leukocytes of the whole blood that are nucleic acid-containing cells are captured by the carrier.

The size of the carrier is not particularly limited as described above, and can be decided suitably depending on the amount of the whole blood to be treated, and the material and the shape of the carrier, for example. Specific examples will be described below.

In the case where the carrier is a sheet-shaped carrier, a film-shaped carrier, or a block-shaped carrier composed of nonwoven fabric, woven fabric, or a porous body, for example, the whole blood and the carrier are brought into contact with each other under the following conditions. The ratio (X : Y) of the volume (X) of the whole blood treated in step (A) to the volume (Y: including voids) of the carrier is, for example, 1 $2.2 \times 10^{-3}$ to 1 : 0.7, preferably 1 : $2.2 \times 10^{-3}$ to 1 : 0.31, and more preferably 1 : $2.2 \times 10^{-3}$ to 1: 0.13. The ratio (X' : Y) of the volume (X') of the treatment liquid containing the whole blood from step (A) to the volume (Y: including voids) of the carrier is, for example, 1 : $1.1 \times 10^{-3}$ to 1 : 0.35, preferably 1 : $1.1 \times 10^{-3}$ to 1 : 0.16, and more preferably 1 : $1.1 \times 10^{-3}$ to 1 : 0.07.

In the case where the carrier is, for example, a bead carrier, use of a plurality of beads is preferable. Examples of the shape of the bead include, but are not limited to, a true sphere, a nearly true sphere, and an oval sphere. The average diameter of the beads is, for example, 30 nm to 100 $\mu$m, preferably 30 nm to 20 $\mu$m, and more preferably 30 nm to 5 $\mu$m. In the case where nucleic acid is recovered from 50 $\mu$L of the whole blood, for example, the total surface area (Z) of the plurality of beads is, for example, 5 $cm^2$ to 2000 $cm^2$, preferably 10 $cm^2$ to 2000 $cm^2$, and more preferably 200 $cm^2$ to 2000 $cm^2$. The filling fraction of the beads is, for example, 0.1% to 10%, preferably 0.1% to 5%, and more preferably 1% to 5%.

Conditions for capturing the nucleic acid complexes by the carrier by bringing the treatment liquid into contact with the carrier are as follows: the treatment temperature is, for example, 15°C to 40°C, preferably 20°C to 35°C, and particularly preferably room temperature (about 25°C); and the treatment time is, for example, 1 second to 10 minutes and preferably 30 seconds to 5 minutes. However, the conditions are not particularly limited thereto.

The method for bringing the treatment liquid into contact with the carrier is not particularly limited, and can be decided suitably depending on the shape of the carrier, for example. The treatment liquid and the carrier can be brought into contact with each other, for example, in a container such as an eppendorf tube, a test tube, or a chip provided with a passage. Specifically, the treatment liquid and the carrier may be brought into contact with each other by adding the carrier and the treatment liquid in the container, or the treatment liquid and the carrier may be brought into contact with each other by adding the treatment liquid in the container in which the carrier is preliminarily fixed.

As will be described below, for example, the treatment liquid and the carrier may be brought into contact with each other by arranging the carrier in a passage of a chip or the like and then passing the treatment liquid through the inside of the carrier arranged on the passage. According to such a method, nucleic acid complexes in the treatment liquid can be held in the carrier and other components can be removed. Here, "passing through the carrier" means that the treatment liquid enters the inside of the carrier and moves inside the carrier, for example. In particular, preferably, the whole or some of the treatment liquid supplied to the carrier enters the inside of the carrier and then passes through the carrier. By causing the treatment liquid to be passed through the carrier in this manner, for example, the amount of undesired components in the treatment liquid remaining in the carrier can be reduced. Thereby, the amount of the undesired components in the nucleic acid sample finally obtained can further be reduced. Further, for example, the treatment liquid and the carrier may be brought into contact with each other by arranging a plurality of the carriers (for example, beads) in a passage of a chip or the like and then passing the treatment liquid through spaces between the carriers. Also according to this method, in the same manner as described above, the nucleic acid complexes can be held in the carriers and other components can be removed. The treatment liquid can be passed through the carriers arranged in the passage or passed through spaces between the carriers arranged in the passage, for example, by reducing or applying pressure.

Separation Step

Subsequently, the carrier is separated from the treatment liquid. The method for separating the carrier from the treatment liquid is not particularly limited and can be decided suitably depending on the shape, arrangement, or the like of the carrier. In the case where the carrier is not fixed in a container, for example, the carrier can be separated from the treatment liquid by taking out the carrier or the treatment liquid from the container. Further, in the case where the carrier is a magnetic bead as described above, the carrier can be separated from the treatment liquid, for example, by recovering the bead with a magnet or the like. On the other hand, in the case where the carrier is fixed in a container, for example, the carrier can be separated from the treatment liquid by removing (draining) the treatment liquid from the container. Further, in the case where the carrier(s) is(are) arranged in a passage as described above, by causing the treatment liquid to be passed through the carrier(s) or by causing the treatment liquid to be passed through spaces between the carriers, for example, the carrier can be separated from the treatment liquid as well as nucleic acid complexes released from blood cells into the treatment liquid can be captured by the carrier(s).

Dispersion Medium-Addition Step

Next, a dispersion medium is added to the carrier separated from the treatment liquid. The dispersion medium may be added before the next heat treatment step in this manner or added after the heat treatment step.

Preferably, the carrier is immersed in the dispersion medium by adding the dispersion medium. Examples of the dispersion medium include, but are not limited to, water; various buffer solutions; aqueous solvents such as buffered saline; organic solvents; and mixtures thereof. Examples of the buffer solutions include Tris-HCl, BES, MOPS, TES, HEPES, DIPSO, TAPSO, POPSO, HEPPSO, EPPS, Tricine, Bicine, and TAPS. The concentration of the buffer solution is, for example, 0.1 mmol/L to 100 mmol/L, preferably 0.1 mmol/L to 50 mmol/L, and particularly preferably 0.1 mmol/L to 10 mmol/L, although it is not particularly limited. The pH of the dispersion medium is, for example, 7.8 to 9.5 and preferably 8.5 to 8.9.

The amount of the dispersion medium to be added is not particularly limited. For example, the ratio of the volume of the whole blood treated in step (A) (X) to the volume of the dispersion medium (D) (volume ratio X: D) is, for example, 1 : 0.5 to 1 : 10, preferably 1 : 0.5 to 1 : 5, and more preferably 1 : 1 top 1: 3.

In advance of the supply of the dispersion medium to the carrier, for example, supply and removal of a washing liquid may be performed. Thereby, undesired components remaining in the carrier can further be removed. The washing liquid is not particularly limited and the examples described for the dispersion medium can be employed. Further, in the case where heat treatment is applied in advance of the supply of the dispersion medium, the supply of the washing liquid to the carrier and the removal of the washing liquid from the carrier are preferably performed in advance of the heat treatment.

Heat Treatment Step

The carrier in the dispersion medium is heat treated. Thereby, nucleic acid is released from the nucleic acid complexes captured by the carrier, and the nucleic acid is released (liberated) in the dispersion medium.

The temperature of the heat treatment is, for example, 50°C to 130°C, preferably 80°C to 130°C, and more preferably 90°C to 100°C, although it is not particularly limited. The heat treatment may be performed at a constant temperature or may be performed with temperature increasing continuously or in a stepwise manner. Further, the treatment time is, for example, 1 minute to 30 minutes and preferably 1 minute to 5 minutes, although it is not particularly limited.

In the case where the heat treatment is performed in advance of the addition of the dispersion medium, by adding the dispersion medium after releasing nucleic acid from the nucleic acid complexes by the heat treatment, the nucleic acid released may be liberated in the dispersion medium.

Subsequently, the dispersion medium after the heat treatment is recovered. As described above, since the nucleic acid is released in the dispersion medium, the dispersion medium can be recovered as a nucleic acid sample that contains whole blood-derived nucleic acid. For example, the nucleic acid sample obtained by the present invention is preferably used for nucleic acid amplification that will be described below, although it is not particularly limited. It is to be noted that, with respect to cell samples other than whole blood, nucleic acid samples can be recovered in the same manner as described above.

According to the method of the present invention, for example, in the case where the cell sample is whole blood, the amount of the whole blood to be treated is 50 μL, the amount of the treatment reagent to be used is 50 μL, and the amount of the dispersion medium to be used is 100 μL, for example, a nucleic acid sample having a concentration of 100 copies/μL to 10000 copies/μL can be recovered. The nucleic acid of this amount is sufficient for use as a template in a nucleic acid amplification method such as PCR, for example. Further, according to the present invention, not only genomic DNA and RNA, but also mitochondrial DNA, which has been difficult to recover, can be recovered.

(Second Embodiment)

In the production method of the present invention, in the case where the cells are eukaryotic cells and the cell sample containing the eukaryotic cells is a sample after refrigeration or freeze-thawing, for example, the second production method as described below is preferred. In the second production method of the present invention, for example, step (A) is a step (A2) of mixing a treatment reagent that does not contain a surfactant but contains protease with the cell sample after freeze-thawing or refrigeration. The treatment reagent used in the second production method of the present invention is also referred to as a "second treatment reagent". Unless otherwise mentioned, the second production method can be performed in the same manner as the first production method except that step (A2) is performed instead of step (A1).

As the cell sample of the present invention, cell samples containing the aforementioned eukaryotic cells can be employed. In the second production method of the present invention, as the second treatment reagent, the treatment reagent that does not contain a surfactant but contains protease can be used as described above. With respect to the cell sample after freeze-thawing or refrigeration, it is presumed that since a part of or the most part of the cell membrane of the eukaryotic cell is broken, for example, breaking of the cell membrane by means of the surfactant can be omitted, and nucleic acid complexes can be released efficiently even with the treatment reagent that does not contain a surfactant. Therefore, the cost of the treatment reagent can further be reduced. However, this presumption does not limit the present invention at all.

Conditions for the freeze-thawing of the cell sample are not limited at all as long as the cell sample is thawed after freezing. The freezing temperature is, for example, -15°C to -200°C and the thawing temperature is, for example, 4°C to 50°C. Further, conditions for the refrigeration of the cell sample are not particularly limited. The refrigeration temperature is, for example, 0°C to 8°C and the storage time is, for example, 4 days to 1 year and may be 1 week to 1 month.

The second treatment reagent may be added to the cell sample before refrigeration or freezing, or the second treatment reagent may be added to the cell sample after refrigeration or freeze-thawing, for example.

The composition of the second treatment reagent is the same as that of the first treatment reagent in the first production method except that the second treatment reagent does not contain a surfactant. Further, in the treatment liquid in which the second treatment reagent and the cell sample are mixed, the amount of the protease relative to the eukaryotic cells and the amount of the protease relative to the cell sample are the same as those in the first production method.

(Third Embodiment)

In the production method of the present invention, in the case where the cells are eukaryotic cells, the cell sample containing the eukaryotic cells is a sample after freeze-thawing, for example, the third production method as described below is preferred. In the third production method of the present invention, for example, step (A) is a step (A3) of mixing a treatment reagent that contains neither a surfactant nor protease with the cell sample after freeze-thawing. The treatment reagent used in the third production method of the present invention is also referred to as a "third treatment reagent". Unless otherwise mentioned, the third production method can be performed in the same manner as the first production method or the second production method except that step (A3) is performed instead of step (A1) or (A2). Further, in the third production method, for example, the cell sample after freeze-thawing may be brought into contact with the carrier in step (B) without mixing with the third treatment reagent in step (A).

As the cell sample of the present invention, cell samples containing the aforementioned eukaryotic cells can be employed. In the third production method of the present invention, as the third treatment reagent, the treatment reagent that contains neither a surfactant nor protease can be used as described above. With respect to the cell sample after freeze-thawing, it is presumed that a part of or the most part of the cell membrane of the eukaryotic cell and a part of or the most part of the nuclear membrane of the eukaryotic cell are broken and, at the same time, proteins packaging nucleic acid in nuclei are denatured in some of the cells and the proteins lose their function of packaging, and thereby nucleic acid complexes are released, for example. Therefore, nucleic acid complexes can be released efficiently even with the treatment reagent that contains neither a surfactant nor protease. Therefore, the cost of the treatment reagent can further be reduced.

Conditions for the freeze-thawing of the cell sample are not limited at all as long as the cell sample is thawed after freezing. The freezing temperature is, for example, -15°C to -200°C and the thawing temperature is, for example, 4°C to 50°C.

The third treatment reagent may be added to the cell sample before freezing, or the third treatment reagent may be added to the cell sample after freeze-thawing, for example.

The composition of the third treatment reagent is, for example, the same as that of the first treatment reagent in the first production method except that the third treatment reagent contains neither a surfactant nor protease. Examples of the third treatment reagent include the various solvents described above.

(Fourth Embodiment)

In the production method of the present invention, in the case where the cells are prokaryotic cells and the cell sample containing the prokaryotic cells is a sample after refrigeration or freeze-thawing, for example, the fourth production method as described below is preferred. In the fourth production method of the present invention, step (A) is a step of freeze-thawing or refrigerating the cell sample containing the prokaryotic cells. Unless otherwise mentioned, the fourth production method can be performed in the same manner as one of the first to third production methods except that step (A4) is performed instead of step (A1), (A2) or (A3).

As the cell sample of the present invention, cell samples containing the aforementioned prokaryotic cells can be employed. In the fourth production method, for example, the nucleic acid complexes can be released simply by refrigerating or freeze-thawing the cell sample.

In the fourth production method, for example, as described above, by freeze-thawing or refrigerating the cell sample in step (A), a treatment liquid in which nucleic acid complexes are released from the cells can be obtained. Preferably, the treatment liquid after step (A) is mixed with a treatment reagent in advance of step (B) in which the treatment liquid is brought into contact with the carrier, for example. As the treatment reagent, a reagent that contains neither protease nor a surfactant or a reagent that contains one of protease and a surfactant and does not contain the other can be employed. The treatment reagent used in the fourth production method of the present invention is also referred to as a "fourth treatment reagent". In the case where the fourth treatment reagent contains protease or a surfactant, the amount of the protease or the surfactant to be added is, for example, the same as that in the first treatment reagent in the first production method. Further, the amount of the protease or the surfactant to be added relative to the cell sample is also the same as that in the first production method. In the case where the fourth treatment reagent contains neither protease nor a surfactant, the fourth treatment reagent is, for example, the same as the first treatment reagent in the first production method except that the fourth treatment reagent contains neither protease nor a surfactant. Examples of the fourth treatment reagent include the various solvents described above.

With respect to the cell sample after freeze-thawing or refrigeration, the cell wall of a prokaryotic cell is broken or becomes breakable. Since the prokaryotic cell does not have a cell nucleus, the cell wall thereof is broken or becomes breakable as described above. Therefore, nucleic acid complexes can be released efficiently even with the fourth treatment reagent that contains neither a surfactant nor protease or does not contain one of a surfactant and protease. Therefore, the cost of the treatment reagent can further be reduced.

The fourth treatment reagent may be added to the cell sample before refrigeration or freezing, or the fourth treatment reagent may be added to the cell sample after refrigeration or freeze-thawing.

< Amplification Product Production Method>

As described above, the nucleic acid sample obtained by the nucleic acid sample production method of the present invention can be used as a template sample in various nucleic acid amplification methods, for example. Hence, the present invention is a method for producing a nucleic acid amplification product having a target sequence by a nucleic acid amplification method, comprising the steps of:

   (a) recovering a nucleic acid sample from a cell sample containing cells by the method for producing a nucleic acid sample of the present invention; and
   (b) producing, using nucleic acid in the nucleic acid sample recovered in step (a) as a template, a nucleic acid amplification product having a target sequence in the template by a nucleic acid amplification method. The present invention also may be referred to as a target sequence amplification method.

The nucleic acid amplification product production method of the present invention is characterized in that nucleic acid is recovered by the nucleic acid sample production method of the present invention, and steps and conditions other than this are not limited at all. Further, as described above, according to the nucleic acid sample production method of the present invention, since a sufficient amount of nucleic acid that can be used as a template can be obtained, for example, the nucleic acid recovered in step (a) can directly be used in step (b).
The nucleic acid amplification method in step (b) is not limited at all, and conventionally known amplification methods can be employed. Specific examples thereof include a polymerase chain reaction (PCR) method, a nucleic acid sequence based amplification (NASBA) method, a transcription-mediated amplification (TM) method, a strand displacement amplification (SDA) method, a smart amplification process method, a loop-mediated isothermal amplification (LAMP) method, and an isothermal and chimeric primer-initiated amplification of nucleic acids (ICAN) method. Further, steps and reaction conditions for various nucleic acid amplification methods are not limited and the nucleic acid amplification methods can be performed, for example, according to conventionally known conditions.
The reagent used for the nucleic acid amplification method is not particularly limited and conventionally known reagents can be employed, and examples thereof include polymerase, nucleoside triphosphate (dNTP), buffering agents, and solvents such as water and buffer solutions. Other than these, the reagent may include, for example, glycerol, heparin, betaine, KCl, $MgCl_2$, and $MgSO_4$.

<Nucleic Acid Recovery Reagent>

The first nucleic acid recovery reagent of the present invention is a reagent used for the first nucleic acid sample production method of the present invention and the reagent contains the protease and the surfactant. The first nucleic acid recovery reagent of the present invention is the same as the first treatment reagent in the first nucleic acid sample production method of the present invention, for example. In the nucleic acid recovery reagent, the protease and the surfactant are added such that the amount of the protease relative to $1 \times 10^2$ to $1 \times 10^9$ cells is, for example, 0.02 mU or more, and the amount of the surfactant relative to $1 \times 10^2$ to $1 \times 10^9$ cells is, for example, 1 femtomole or more when the first nucleic acid recovery reagent is mixed with the cell sample.
The first nucleic acid recovery reagent of the present invention is applicable as long as the protease and the surfactant are added to be in the aforementioned range when the first nucleic acid recovery reagent is mixed with the cell sample, for example.
The second nucleic acid recovery reagent of the present invention is a reagent used for the second nucleic acid sample production method of the present invention and is not particularly limited. The second nucleic acid recovery reagent is applicable as long as it contains at least protease. Preferably, the amount of the protease is in the same range as the first treatment reagent when the second nucleic acid recovery reagent is mixed with the cell sample. The second nucleic acid recovery reagent of the present invention is the same as the second treatment reagent in the second nucleic acid sample production method of the present invention, for example. Further, the third nucleic acid recovery reagent of the present invention is a reagent used for the third nucleic acid sample production method of the present invention, and the third nucleic acid recovery reagent may be a solvent that contains one of protease and a surfactant or a solvent that contains neither protease nor a surfactant. The third nucleic acid recovery reagent of the present invention is the same as the third treatment reagent in the third nucleic acid sample production method of the present invention, for example. The fourth nucleic acid recovery reagent of the present invention is a reagent used for the fourth nucleic acid sample production method of the present invention, and the fourth nucleic acid recovery reagent may be a solvent that contains neither protease nor a surfactant or a solvent that contains one of protease and a surfactant and does not contain the other. The fourth nucleic acid recovery reagent of the present invention is the same as the fourth treatment reagent in the fourth nucleic acid sample production method of the present invention, for example.

The nucleic acid recovery reagents of the present invention may be liquid or solid, for example. In the case where the nucleic acid recovery reagents of the present invention are liquid, for example, the stock solutions thereof may be used directly as the aforementioned treatment reagents or the diluents thereof that have been diluted before use may be used as the treatment reagents. In the case where the nucleic acid recovery reagents are solid, the nucleic acid recovery reagents may be dissolved or dispersed in the solvents of the treatment reagents before the nucleic acid recovery reagents are mixed with the cell samples, and the resultants may be used as the aforementioned treatment reagents. The nucleic acid recovery reagents of the present invention may further contain other components, for example. In the case where recovery of nucleic acid from cells and nucleic acid amplification of a target sequence are performed with the nucleic acid recovery reagents of the present invention, for example, the nucleic acid recovery reagents of the present invention may further contain components required for the nucleic acid amplification. Examples of the foregoing components include polymerase, nucleoside triphosphate (dNTP), buffering agents, and solvents such as water and buffer solutions. The nucleic acid recovery reagent of the present invention also can be referred to as a production reagent used for the nucleic acid amplification product production method of the present invention, for example.

<Nucleic Acid Recovery Kit>

The first nucleic acid recovery kit of the present invention is a kit used for the first nucleic acid sample production method of the present invention, and is characterized by including the carrier, the protease, and the surfactant. In the present invention, the carrier, the protease, and the surfactant are as described above.

The first nucleic acid recovery kit of the present invention may include, as the carrier, the nucleic acid recovery chip or nucleic acid amplification chip of the present invention having the carrier that will be described below, for example.

In the first nucleic acid recovery kit of the present invention, the protease and the surfactant may each be contained in a separate container or both may be contained in the same container. In the case where the protease and the surfactant coexist, the first nucleic acid recovery kit of the present invention preferably includes the carrier and the first nucleic acid recovery reagent of the present invention. The first nucleic acid recovery reagent of the present invention is as described above, and is applicable as long as it contains the protease and the surfactant. The first nucleic acid recovery reagent of the present invention may be liquid or solid. In the first nucleic acid recovery kit of the present invention, the amounts of the protease and the surfactant are not particularly limited, and it is applicable as long as the amounts are in the aforementioned ranges at the time of use, for example.

The second nucleic acid recovery kit of the present invention is a kit used for the second nucleic acid sample production method of the present invention, and is not particularly limited. The second nucleic acid recovery kit includes the carrier and the protease. For example, the second nucleic acid recovery kit may include the carrier and the second nucleic acid recovery reagent of the present invention. Further, the third nucleic acid recovery kit of the present invention is a kit used for the third nucleic acid sample production method of the present invention. For example, the third nucleic acid recovery kit may include the carrier and one of protease and a surfactant; the kit may include the carrier and a solvent that contains neither protease nor a surfactant; or the kit may include the carrier and the third nucleic acid recovery reagent of the present invention. The fourth nucleic acid recovery kit of the present invention is a kit used for the fourth nucleic acid sample production method of the present invention. For example, the fourth nucleic acid recovery kit may include the carrier and a solvent that contains neither protease nor a surfactant; the kit may include the carrier and a solvent that contains one of protease and a surfactant and does not contain the other; or the kit may include the carrier and the fourth nucleic acid recovery reagent of the present invention.

The nucleic acid recovery kits of the present invention may each further have, for example, an instruction manual. Further, the nucleic acid recovery kits of the present invention may each further include, for example, other components. In the case where recovery of nucleic acid from cells and nucleic acid amplification of a target sequence are performed with the nucleic acid recovery kits of the present invention, for example, the nucleic acid recovery kits of the present invention may further include components required for the nucleic acid amplification. Examples of the foregoing components include polymerase, nucleoside triphosphate (dNTP), buffering agents, and solvents such as water and buffer solutions. The nucleic acid recovery kit of the present invention can also be referred to as a production kit used for the nucleic acid amplification product production method of the present invention, for example.

< Nucleic Acid Recovery Chip >

The nucleic acid recovery chip of the present invention is a chip used for the nucleic acid sample production method of the present invention, and is characterized by including a chip body and a carrier, wherein the chip body is provided with a liquid passage and the carrier is arranged in the passage. By using the nucleic acid recovery chip of the present invention and the nucleic acid recovery reagent of the present invention, for example, the nucleic acid sample production method of the present invention can be performed.

The nucleic acid recovery chip of the present invention is applicable as long as the carrier is arranged in the passage through which liquids such as the cell sample and the treatment reagent are passed, and the configuration, the shape, and the like except for this are not limited at all. Embodiments of the nucleic acid recovery chip of the present invention will be described below. However, the present invention is not limited thereto.

(First Nucleic Acid Recovery Chip)

The first nucleic acid recovery chip includes, for example, a chip body and a carrier and the chip body includes a liquid passage. One end of the passage is provided with a first opening for introducing and draining a liquid and the other end of the passage is provided with a second opening that is connectable to pressure control means, and the carrier is arranged in the passage. The carrier may be a sheet-shaped carrier that can be used as a filter or a plurality of bead-shaped carriers.

With respect to the first nucleic acid recovery chip, for example, at the time of using, by connecting the pressure control means to the second opening and controlling the passage to be under a pressure-reduced condition by the pressure control means, the liquid can be introduced into the passage from the first opening; and by controlling the passage to be under a pressurized condition by the pressure control means, the liquid introduced can be drained to the outside of the passage from the first opening. Therefore, first, when the treatment liquid containing the cell sample is introduced into the passage under the pressure-reduced condition, the treatment liquid passes through the carrier arranged in the passage. Thereby, nucleic acid complexes released from cells in the cell sample by means of the treatment reagent are captured by the carrier when passing through the passage, for example. Subsequently, when the treatment liquid introduced is drained to the outside of the passage under the pressurized condition, undesired components derived from the cell sample can be removed. Further, when the carrier is applied with heat treatment in the presence of the dispersion medium after introducing the dispersion medium into the passage under the pressure-reduced condition, nucleic acid is released into the dispersion medium from the nucleic acid complexes captured by the carrier. Hence, by draining the dispersion medium to the outside of the passage under the pressurized condition, the dispersion medium containing the nucleic acid can be recovered as a nucleic acid sample. The nucleic acid recovery chip and the pressure control means may directly or indirectly be connected. It is to be noted that the method for introducing the cell sample and the treatment reagent is not particularly limited. For example, a treatment liquid in which the cell sample and the treatment reagent are mixed may preliminarily be provided and this may be introduced into the passage; an operation (pipetting) of introducing one of the cell sample and the treatment reagent and draining it to the other may be repeated and finally the treatment liquid may be introduced.

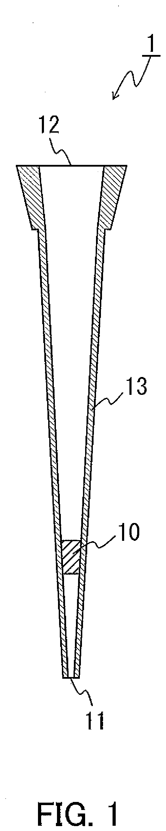

As a nucleic acid recovery chip of such an embodiment, for example, a pipette chip that is used after attaching on a suction device can be employed. In this case, for example, a suction device such as a manual or automatic pipetter serves as the pressure control means. The first nucleic acid recovery chip will be described with reference to a pipette chip as an example using FIG. 1. FIG. 1 is a cross-sectional view of a nucleic acid recovery pipette chip (hereinafter, referred to as a "pipette chip"). As shown in FIG. 1, a pipette chip 1 is provided with a hollow-cone-shaped body 13; a first opening 11, which is a drain port of a liquid; and a second opening 12, which is attachable to a pipetter. A carrier 10 is arranged in the cone-shaped body 13 at the first opening 11 side. In the pipette chip 1, a void in the cone-shaped body 13 serves as a liquid passage and a container for a liquid introduced therein.

The size and the shape of the pipette chip are not particularly limited and can be decided suitably depending on the amount of the treatment liquid containing the cell sample to be introduced, for example. Since the nucleic acid recovery chip of the present invention is characterized by including the carrier as described above, for example, the nucleic acid recovery chip can be produced simply by arranging the carrier in a commercially available pipette chip. Further, attachment of the pipette chip to a suction device such as a pipette can be performed in the same manner as a conventional method.

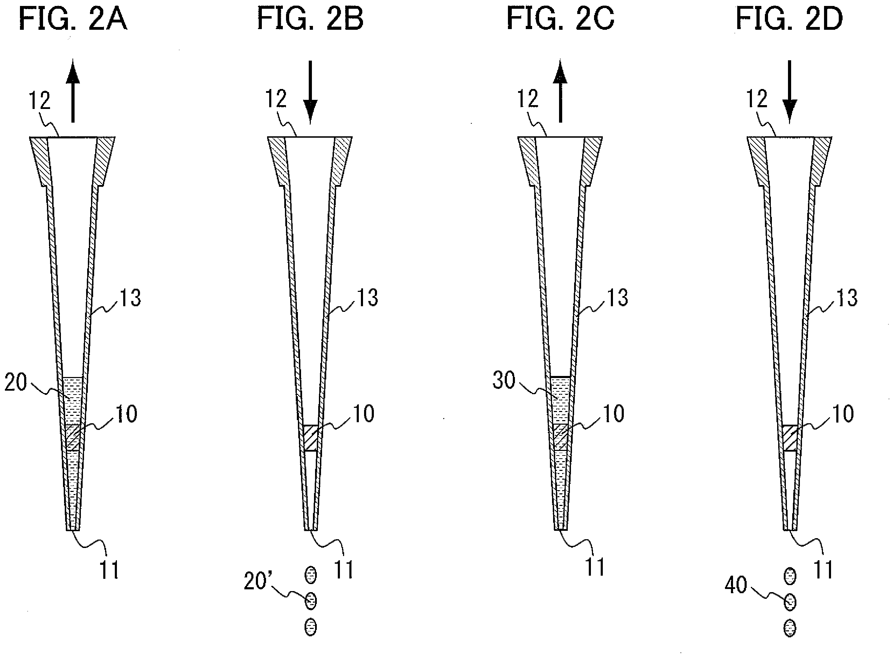

An example of the nucleic acid sample production method (nucleic acid recovery method) using the pipette chip 1 will be described using FIGs. 2A to 2D. However, the present invention is not limited thereto. FIGs. 2A to 2D are cross-sectional views showing the outline of the step of the nucleic acid sample production method using the pipette chip. In FIGs. 2A to 2D, identical parts to those shown in FIG.1 are indicated with identical numerals.

First, the pipette chip 1 is attached to a pipetter (not shown) from the second opening 12 side. Then, as shown in FIG. 2A, a treatment liquid 20 is sucked from the first opening 11 by means of an operation of the pipetter. At that time, preferably, all of the sucked treatment liquid passes through the carrier 10 in the pipette chip 1. Thereby, nucleic acid complexes released in the treatment liquid 20 are captured by the carrier 10. Next, as shown in FIG. 2B, the treatment liquid 20 which has been sucked in is drained to the outside from the first opening 11 by means of an operation of the pipetter. A treatment liquid 20' drained here is a liquid in which nucleic acid complexes are removed by the carrier 10 and is a liquid containing undesired components for nucleic acid recovery such as erythrocyte. Subsequently, as shown in FIG. 2C, a dispersion medium 30 is sucked from the first opening 11 by means of an operation of the pipetter. At that time, preferably, the carrier 10 in the pipette chip 1 is filled with the dispersion medium 30 which has been sucked in. Further, in this state, i.e., in the state where the carrier 10 is present in the dispersion medium 30, the heat treatment as described above is performed. Finally, as shown in FIG. 2D, a dispersion medium 40 after the heat treatment is drained to the outside from the first opening 11 by means of an operation of the pipetter. The drained dispersion medium 40 contains nucleic acid released from the nucleic acid complexes that have been captured by the carrier 10 as described above. When the dispersion medium 40 is recovered as a nucleic acid sample, it can be used as a template sample for nucleic acid amplification, for example. With respect to this nucleic acid sample, since undesired components for nucleic acid recovery are drained in the step represented by FIG. 2B, the amount of undesired components is reduced.

(Second Nucleic Acid Recovery Chip)

The second nucleic acid recovery chip includes, for example, the chip body and the carrier and the chip body includes a liquid passage. One end of the passage is provided with a first opening for introducing a liquid that is connectable to pressurizing means and the other end of the passage is provided with a second opening for draining the liquid, and the carrier for nucleic acid recovery of the present invention is arranged in the passage.

With respect to the second nucleic acid recovery chip, at the time of using, by connecting the pressurizing means to the first opening and pressurizing the liquid by the pressurizing means, the liquid can be introduced into the passage from the first opening; and by pressurizing the passage by the pressurizing means, the liquid introduced can be drained to the outside of the passage from the second opening. Therefore, first, when the treatment liquid is introduced into the passage under the pressurized condition, the treatment liquid passes through the carrier arranged in the passage, and therefore nucleic acid complexes in the treatment liquid are captured by the carrier. Subsequently, when the treatment liquid introduced is drained to the outside of the passage under the pressurized condition, undesired components derived from the cell sample can be removed. Further, when the carrier is applied with heat treatment in the presence of the dispersion medium after introducing the dispersion medium into the passage under the pressurized condition, nucleic acid is released into the dispersion medium from the nucleic acid complexes captured by the carrier. Hence, by draining the dispersion medium to the outside of the passage under the pressurized condition, the dispersion medium containing the nucleic acid can be recovered as a nucleic acid sample. The nucleic acid recovery chip and the pressurizing means may directly or indirectly be connected (hereinafter, the same applies).

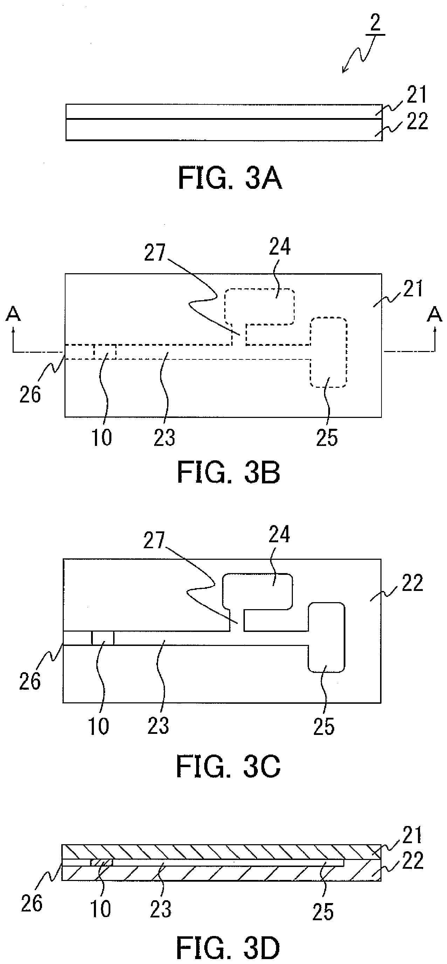

As a nucleic acid recovery chip of such an embodiment, for example, a so-called biochip formed of a support substrate having a concave passage formed on the surface thereof and a cover substrate with which the surface of the support substrate is covered can be employed. The second nucleic acid recovery chip will be described with reference to a biochip as an example using FIGs. 3A to 3D. FIGs. 3A to 3D are schematic views showing a nucleic acid recovery biochip (hereinafter, referred to as a "biochip"); and FIG. 3A is a side view, FIG 3B is a top view, and FIG. 3D is a cross-sectional view taken along the line A-A of FIG. 3B. FIG. 3C is a top view of a support substrate 22 of the biochip. As shown in FIGs. 3A to 3D, a biochip 2 includes a support substrate 22 and a cover substrate 21. A passage 23, a passage 27 branched from the passage 23, a drainage portion 24 connected to the passage 27, and a storage portion 25 for a nucleic acid-containing liquid (nucleic acid sample) connected to the passage 23 are formed on the surface of the support substrate 22 as concave grooves. The surface of the support substrate 22 on which the concave grooves are formed is covered with the cover substrate 21, and thereby an end of the passage 23 (the left end in FIGs. 3B to 3D) serves as an inlet port 26 for the liquid. A carrier 10 is arranged in the passage 23 at the inlet port 26 side relative to a connection site with the drainage portion 24 and a connection site with the storage portion 25. Although the passage 23 is connected to the storage portion 25 of the nucleic acid sample and the drainage portion 24 through the passage 27, introduction of the liquid to the drainage portion 24 and the introduction of the liquid to the storage portion 25 can be switched suitably. The size and the shape of the biochip are not particularly limited.

The nucleic acid recovery method using the biochip 2 will be described with reference to a method using a liquid-pressure-feeding device such as a syringe as an example using FIGS. 3A to 3D. However, the present invention is not limited thereto.

A treatment liquid is fed to the inlet port 26 by means of the liquid-pressure-feeding device (not shown). Thereby, the fed treatment liquid is passed through the carrier 10 arranged in the passage 23 and stored in the drainage portion 24 through the passage 27. Then, nucleic acid complexes released in the treatment liquid are captured by the carrier 10. Here, the treatment liquid stored in the drainage portion 24 is a liquid in which nucleic acid complexes are removed by the carrier 10 and a liquid containing undesired components for nucleic acid recovery such as erythrocytes. Next, in the same manner, a dispersion medium is fed to inlet port 26 by means of the liquid-pressure-feeding device. At that time, preferably, the carrier 10 in the passage 23 is filled with the dispersion medium. Further, in this state, i.e., in the state where the carrier 10 is present in the dispersion medium, the heat treatment as described above is performed. Finally, the dispersion medium after the heat treatment is transferred to the storage portion 25 of a nucleic acid-containing liquid by means of the liquid-pressure-feeding device. The dispersion medium stored in the storage portion 25 contains nucleic acid released from the nucleic acid complexes that have been captured as described above. The dispersion medium that contains the nucleic acid, as a nucleic acid sample, can be used as a template sample for nucleic acid amplification, for example. With respect to this nucleic acid sample, since undesired components for nucleic acid recovery are drained, the amount of the undesired components is reduced.

The aforementioned method is an example of a method for introducing and draining the liquid by pressurizing. However, the present invention is not limited thereto. That is, for example, (i) the liquid can be introduced and drained by reducing pressure; (ii) the liquid can be introduced by applying pressure and drained by reducing pressure; or (iii) the liquid can be introduced by reducing pressure and drained by applying pressure. With respect to the nucleic acid recovery chip used for the method (i), for example, preferably, the chip body is provided with a first opening for introducing a liquid at one end of the passage and is provided with a second opening for draining the liquid that is connectable to pressure reducing means at the other end of the passage. In this nucleic acid recovery chip, for example, at the time of using, preferably, the liquid is introduced into the passage from the first opening and the liquid introduced is drained to the outside of the passage from the second opening by connecting the pressure reducing means to the second opening and reducing the pressure in the passage by the pressure reducing means. The nucleic acid recovery chip and the pressure reducing means may directly or indirectly be connected (hereinafter, the same applies). With respect to the nucleic acid recovery chip used for the method (ii), for example, preferably, the chip body is provided with a first opening for introducing a liquid that is connectable to pressurizing means at one end of the passage and is provided with a second opening for draining the liquid that is connectable to pressure reducing means at the other end of the passage. In this nucleic acid recovery chip, for example, at the time of using, preferably, the liquid is introduced into the passage from the first opening by connecting the pressurizing means to the first opening and pressurizing the liquid by the pressurizing means, and the liquid introduced is drained to the outside of the passage from the second opening by connecting the pressure reducing means to the second opening and reducing the pressure in the passage by the pressure reducing means. With respect to the nucleic acid recovery chip used for the method (iii), for example, preferably, the chip body is provided with a first opening for introducing a liquid that is connectable to pressurizing means at one end of the passage and is provided with a second opening for draining the liquid that is connectable to pressure reducing means at the other end of the passage. In this nucleic acid recovery chip, at the time of using, preferably, the liquid is introduced into the passage from the first opening by connecting the pressure reducing means to the second opening and reducing the pressure in the passage by the pressure reducing means, and the liquid introduced is drained to the outside of the passage from the second opening by connecting the pressurizing means to the first opening and by pressurizing the passage by the pressurizing means. These embodiments can be set suitably depending on the combination, for example, by allowing the first opening for introducing the liquid to be connectable to the pressurizing means or by allowing the second opening for draining the liquid to be connectable to the pressure reducing means.

As the carrier, for example, a plurality of bead-shaped carriers also can be employed. In this case, for example, the passage is filled with the bead-shaped carriers. In this embodiment, for example, by feeding the treatment liquid to the passage, the treatment liquid is passed through spaces between the bead-shaped carriers, and at that time, nucleic acid complexes in the treatment liquid are captured by the bead-shaped carriers at the surfaces thereof. Further, in the case where the bead-shaped carriers are porous bodies, for example, the nucleic acid complexes can be captured not only at the outer surfaces but also at the exposed surfaces in voids.

The nucleic acid recovery biochip of the present invention may further include a reaction portion for performing various reactions using nucleic acid; a detection portion; and a reagent portion for storing a reagent required for the reaction. Specifically, in the nucleic acid recovery biochip, the storage portion may be connected to the reaction portion through the passage or the reaction portion may be connected to the reagent portion and the detection portion through the passage. Further, a reagent required for the desired reaction may preliminarily be arranged in the storage portion or the storage portion may be connected to the reagent portion through the passage. In such an embodiment, for example, the storage portion serves as the reaction portion. When the reaction portion, the detection portion, and the like are connected in this manner, for example, not only recovery of nucleic acid from a cell sample but also a desired reaction using the nucleic acid recovered and measurement of the reaction result can be performed in one biochip. Therefore, by applying the present invention to various analyses using nucleic acid such as nucleic acid amplification, SNP detection, and the like, a simple analysis as compared to a conventional method can be performed. Further, since the nucleic acid recovery chip of the present invention is applicable as long as the carrier is arranged in the passage, the size of the biochip can be reduced. As a further embodiment of such a biochip, a nucleic acid amplification chip will be described below as an example.

< Nucleic Acid Amplification Chip >

The nucleic acid amplification chip of the present invention is a chip used for the method for producing a nucleic acid amplification product of the present invention, comprising:

a chip body comprising:

an opening for introducing a liquid;
a drainage portion for storing drainage;
a reaction portion for performing a nucleic acid amplification reaction; and
a liquid passage; and

a carrier, wherein

an end of the passage is the opening,
the passage is connected to the drainage portion and the reaction portion, and the carrier is arranged in the passage at the opening side relative to a connection site with the drainage portion and a connection site with the reaction portion. By using the nucleic acid amplification chip of the present invention and the nucleic acid recovery reagent of the present invention, for example, a nucleic acid amplification product production method using the nucleic acid sample production method of the present invention can be performed.

The nucleic acid amplification chip of the present invention is applicable as long as the passage through which liquids such as the treatment liquid, the cell sample, and the treatment reagent are passed is provided with the carrier, and the configuration and the shape except for this are not limited at all.

In addition to the passage (first passage), the nucleic acid amplification chip of the present invention further includes a second passage and a third passage, the reaction portion and the first passage may directly or indirectly be connected through the second passage, and the drainage portion and the first passage may directly or indirectly be connected through the third passage. Further, with respect to the nucleic acid amplification chip of the present invention, for example, preferably, introduction of the liquid to the drainage portion and introduction of the liquid to the reaction portion are switchable. Specifically, further preferably, the introduction of the liquid to the drainage portion and the introduction of the liquid to the reaction portion are switchable because introduction of the liquid to the second passage and introduction of the liquid to the third passage are switchable, for example.

In the present invention, treatment required for the release of nucleic acid from the nucleic acid complexes captured by the carrier and treatment required for the amplification reaction are both heat treatment. Therefore, with respect to the nucleic acid amplification chip of the present invention, for example, by simply attaching the nucleic acid amplification chip to a device provided with temperature control means and performing temperature control, the recovery of the nucleic acid and the amplification reaction can be performed.

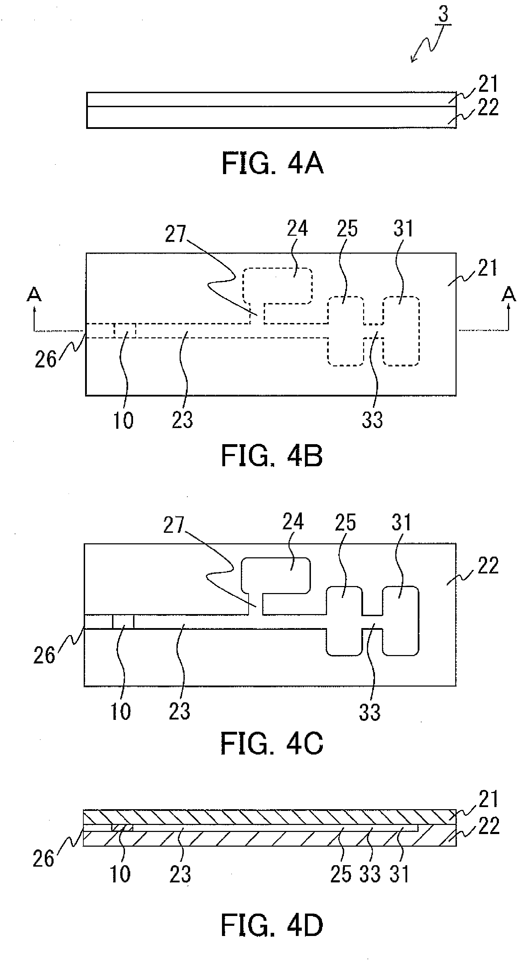

As a nucleic acid amplification chip of such an embodiment, for example, a so-called biochip formed of a support substrate having a concave passage formed on the surface thereof and a cover substrate with which the surface of the support substrate is covered can be employed. The nucleic acid amplification chip of the present invention will be described with reference to a biochip as an example using FIGs. 4A to 4D. Unless otherwise mentioned, the embodiment of the biochip shown in FIGs. 4A to 4D is the same as that of the biochip shown in FIGs. 3A to 3D and the biochip shown in FIGs. 4A to 4D can be used in the same manner as the biochip shown in FIGs. 3A to 3D.

FIGs. 4A to 4D are schematic views showing a nucleic acid amplification biochip (hereinafter, referred to as a "biochip"); and FIG. 4A is a side view, FIG 4B is a top view, and FIG. 4D is a cross-sectional view taken along the line A-A of FIG. 4B. FIG. 4C is a top view of a support substrate 22 of the biochip. In Figs. 4A to 4D, identical parts to those shown in FIGs.3A to 3D are indicated with identical numerals. As shown in FIGs. 4A to 4D, a biochip 3 includes the support substrate 22 and a cover substrate 21. A first passage 23, a third passage 27 connected to the first passage 23, a drainage portion 24 connected to the third passage 27, a storage portion 25 for a nucleic acid-containing liquid (nucleic acid sample) connected to the first passage 23, a second passage 33 connected to the storage portion 25, and a reaction portion 31 connected to the second passage 33 are formed on the surface of the support substrate 22 as concave grooves. The surface of the support substrate 22 on which the concave grooves are formed is covered with the cover substrate 21, and thereby an end of the passage 23 (the left end in FIGs. 4B to 4D) serves as an inlet port 26 for the liquid. A carrier 10 is arranged in the passage 23 at the inlet port 26 side relative to a connection site with the storage portion 25 and a connection site with the drainage portion 24 (connection site with the third passage 27). Although the passage 23 is connected to the storage portion 25 of the nucleic acid-containing liquid and the drainage portion 24 through the third passage 27, introduction of the liquid to the drainage portion 24 and introduction of the liquid to the storage portion 25 can be switched suitably.

In the biochip 3, the nucleic acid-containing liquid stored in the storage portion 25 is further transferred to the reaction portion 31 through the second passage 33, for example. Then, in the case where a reagent required for an amplification reaction is present in the reaction portion 31, the amplification reaction can be started without performing any further operation. Further, in the case where the biochip 3 includes a reagent portion (not shown) storing the reagent separately, for example, after feeding the reagent in the reagent portion to the reaction portion 31, the amplification reaction can be started. The detection of the amplification reaction can be performed in the reaction portion 31, for example. In the case where the biochip 3 further includes a detection portion (not shown), for example, after feeding the reaction solution from the reaction portion 31 to the detection portion, the detection may be performed in the detection portion.

Examples

Next, Examples of the present invention will be described. However, the present invention is not limited by the Examples.

Example 1

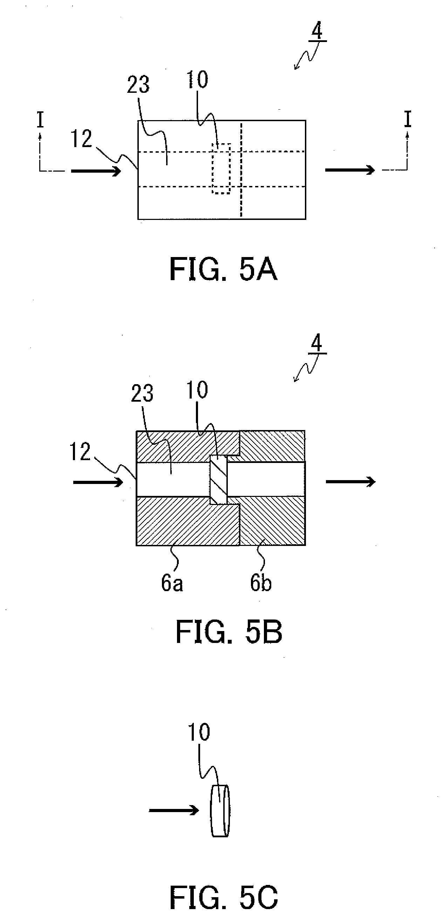

Each of the carriers (1a and 1b) in the following table 2 were cut into a circle having a diameter of 2 mm, and this was attached to a jig as shown in FIGs. 5A to 5C. FIGs. 5A to 5C are schematic views showing a jig to which a carrier is attached. FIG. 5A is a top view of the jig to which the carrier is attached, FIG. 5B is a cross-sectional view taken along the line I to I of FIG. 5A, and FIG. 5C is a schematic view indicating the direction of a liquid passing through a carrier. A jig 4 includes a passage 23 and a carrier 10, and the carrier 10 is arranged in the passage 23 in the vicinity of the center thereof in the axial direction. Specifically, in the jig 4, the carrier 10 is arranged at a concave portion of a concave member 6a made of aluminum, and by fitting a convex portion of a convex member 6b made of aluminum to the concave portion, the carrier 10 is fixed in an aluminum block formed of the concave member 6a and the convex member 6b. Although it is not shown, the aluminum block is fixed with a plastic member such that the bottom surface (lower surface in FIG. 5B) thereof is exposed. This jig 4 was arranged on a heating block such that the exposed bottom surface was brought into contact with the heating block. In each of the Figs. 5A to 5C, the arrow indicates the direction of the liquid. As indicated by the arrow in FIG. 5C, in Example 1, the liquid is introduced from the circular surface of the carrier 10 and drained from the other surface.

50 $\mu$L of whole blood and 50 $\mu$L of the treatment reagent 1-1 in the following table 1 were mixed to prepare 100 l1L of a treatment liquid. In the treatment liquid, relative to 50 $\mu$L of whole blood, the amount of protease was 5.4 U and the amount of surfactant was 0.25 vol%. 100 $\mu$L of the treatment liquid was introduced into the passage 23 of the jig 4 from a first opening of the passage 23 by pressurizing, passed through the carrier 10, and drained from a second opening of the passage 23. Then, 100 $\mu$L of the washing liquid in the following table 1 was introduced into the passage 23 in the same manner as described above, passed through the carrier 10, and drained from the passage 23. After heating the carrier 10 in the jig 4 at 96°C for 5 minutes by the heating block, 100 $\mu$L of the dispersion medium in the following table 1 was introduced into the passage 23 in the same manner as described above, passed through the carrier 10, and drained from the passage 23. The dispersion medium drained was used as a nucleic acid sample.

### Table 1

| (Treatment Reagent 1-1) | |
|---|---|
| Urea | 1.5 mol/L |
| Proteinase K | 4 $\mu$g (108 mU/$\mu$L |
| Tris-HCl (pH 8.9) | 20 mmol/L |
| Nonidet® P-40 | 0.5% |
| (Washing liquid and dispersion medium) | |
| Tris-HCl (pH 8.6) | 1 mmol/L |
| EDTA | 0.1 mmol/L |

### Table 2

| Example | Polymer | Material | Properties | Number of cells in 50 $\mu$L of whole blood (number of pieces) | DNA concentration (copy/$\mu$L) |
|---|---|---|---|---|---|
| 1a | Nylon | Woven fabric (Mesh type) | Thickness: 70 $\mu$m  Mesh size: 100 $\mu$m | about 295,000 | 2230 (recovery rate: 38%) |

(continued)

| Example | Polymer | Material | Properties | Number of cells in 50 μL of whole blood (number of pieces) | DNA concentration (copy/μL) |
|---|---|---|---|---|---|
| 1b | Polyurethane (Polyester type) | Porous body (Sponge) | Thickness: 2 mm Density: 80±10 kg/m³ | about 285,000 | 684 (recovery rate: 12%) |

1a: nylon (product name), produced by Sefar AG
1b: MF80A (product name), produced by INOAC CORPORATION

With respect to each of the nucleic acid samples recovered, the concentration of genomic DNA was measured by the real time PCR method. Further, the theoretical value of the genomic DNA amount in 50 μL of whole blood was calculated and the recovery rate of the genomic DNA by each of the carriers was calculated according to the following formula. The results thereof are summerized in table 2.

$$\text{Recovery rate (\%)} = 100 \times (A \times B) / C$$

A: DNA concentration of nucleic acid sample
B: Volume of nucleic acid sample
C: Theoretical value of DNA amount in 50 μL of whole blood

As summarized in table 2, according to the method of Example 1, different from a conventional method, for example, genomic DNA could be recovered simply without using a special agent. Further, it was confirmed that the concentration of genomic DNA in each of the nucleic acid samples recovered was more than 500 copies/μL, which was a requisite amount for PCR and the like.

Example 2

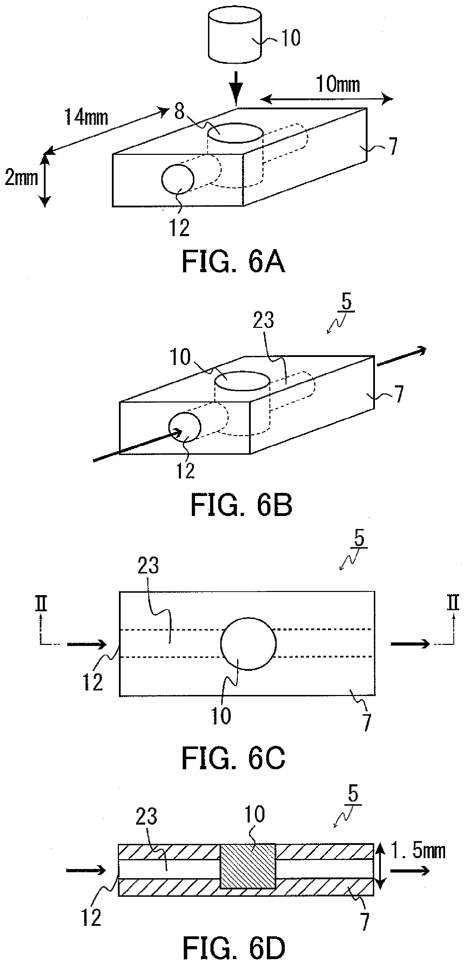

Carriers (2a to 2j) in the following table 3 were provided. Each of the carriers 2b (woven fabric), 2c (woven fabric), and 2j (nonwoven fabric) was cut into circles each having a diameter of 2 mm, and the circular carriers were laminated to form a cylindrical carrier having a thickness of 1.5 mm. Each of the rest of carriers was cut into a cylinder having a diameter of 2 mm and a height of 1.5 mm. The laminates, 2b, 2c, and 2j, and the monolayers were each attached to a jig as shown in FIGs. 6A to 6D as a cylindrical carrier. FIGs. 6A and 6D are schematic views showing a jig to which a carrier is attached. FIG. 6A is a perspective view showing the jig body and the carrier, FIG. 6B is a perspective view of the jig to which the carrier is attached, FIG. 6C is a top view of the FIG. 6B, and FIG. 6D is a cross-sectional view taken along the line II to II of FIG. 6C. A jig 5 is provided with a main body 7 including a passage 23 having a diameter of 1 mm and a carrier arrangement portion 8 and a carrier 10. The carrier arrangement portion 8 is provided in the passage 23 in the vicinity of the center thereof in the axial direction, and the carrier 10 is arranged in the carrier arrangement portion 8. Although it is not shown, the exposed surface of the carrier 10 (upper surface in FIG. 6D) is covered with an adhesive sheet. In Example 2, as indicated by the arrows in FIGs. 6B to 6D, each liquid is introduced from a first side surface of the cylindrical carrier and drained from a second side surface.
50 μL of whole blood and 50 μL of the treatment reagent 1-1 of Example 1 were mixed to prepare 100 μL of a treatment liquid. Then, 100 μL of the treatment liquid was introduced into the passage 23 of the jig 5 from a first opening of the passage 23 by pressurizing, passed through the carrier 10, and drained from a second opening of the passage 23. Next, 100 μL of the washing liquid of Example 1 was introduced into the passage 23 in the same manner as described above, passed through the carrier 10, and drained from the passage 23. Then, the carrier 10 was taken out from the jig 5 and was immersed in 100 μL of the dispersion medium of Example 1. Each of the carriers was heated at 96°C for 5 minutes. The dispersion medium after heating was recovered and was used as a nucleic acid sample.

Table 3

| Example | Polymer | Material | Properties | Number of cells in 50 μL of whole blood (number of pieces) | DNAconcentration (copy/μL) |
|---------|---------|----------|------------|------------|------------|
| 2a | PE | Porous body | Pore size: 150 μm Thickness: 2 mm Porosity: 80 % | About 280,000 | 4100 (recovery rate: 73%) |
| 2b | Polyester | Woven fabric (Mesh type) | Thickness: 125 μm Mesh size: 100 μm | About 280,000 | 5010 (recovery rate: 89%) |
| 2c | Nylon | Woven fabric (Mesh type) | Thickness: 95 μm Mesh size: 150 μm | About 280,000 | 4690 (recovery rate: 84%) |
| 2d | Polyurethane (Polyether type) | Porous body (Sponge) | Thickness: 2 mm Density: $75 \pm 10$ kg/m$^3$ | About 280,000 | 3970 (recovery rate: 71%) |
| 2e | Polyurethane (Polyester type) | Porous body (Sponge) | Thickness: 2 mm Density: $30 \pm 5$ kg/m$^3$ | About 295,000 | 3740 (recovery rate: 63%) |
| 2f | Polyurethane (Polyester type) | Porous body (Sponge) | Thickness: 2 mm Density: $80 \pm 10$ kg/m$^3$ | About 350,000 | 4150 (recovery rate: 59%) |
| 2g | Urethane foam | Porous body (Sponge) | Thickness: 2 mm Density: $70 \pm 5$ kg/m$^3$ | About 350,000 | 4120 (Recovery rate: 59%) |
| 2h | Urethane foam | Porous body (Sponge) | Thickness: 2 mm Density: $70 \pm 5$ kg/m$^3$ | About 350,000 | 5880 (Recovery rate: 84%) |
| 2i | Urethane foam | Porous body (Sponge) | Thickness: 2 mm Density: $75 \pm 7$ kg/m$^3$ | About 350,000 | 4520 (Recovery rate: 65%) |

(continued)

| Example | Polymer | Material | Properties | Number of cells in 50 μL of whole blood (number of pieces) | DNAconcentration (copy/μL) |
|---|---|---|---|---|---|
| 2j | PP | Nonwoven fabric | Thickness: 170 μm Thread diameter: About 2 μm Basis weight: 20 g/m$^3$ | About 350,000 | 3830 (Recovery rate: 58%) |

2a: MAPS (product name), produced by INOAC CORPORATION
2b: PET150-HD (product name), produced by Sefar AG
2c: 9P-150 (product name), produced by Sefar AG
2d: CFS (product name), produced by INOAC CORPORATION
2e: MF50 (product name), produced by INOAC CORPORATION
2f: MF80 (product name), produced by INOAC CORPORATION
2g: 800EA (product name), produced by KURABO INDUSTRIES LTD.
2h: 806EA (product name), produced by KURABO INDUSTRIES LTD.
2i:: 852EA (product name), produced by KURABO INDUSTRIES LTD.
2j: EM02010 (product name), produced by Toray Fine Chemicals Co., Ltd.
PP: polypropylene
PE: polyethylene

With respect to each of the nucleic acid samples recovered, the concentration of genomic DNA was measured in the same manner as in Example 1, and the recovery rate of the genomic DNA by each of the carriers was calculated. The results thereof are summarized in table 3.

With respect to the nucleic acid sample of Example 2a, the concentration of mitochondrial DNA was measured by the real time PCR method. The result was about $10^5$ copies/μL.

As summarized in table 3, according to the method of Example 2, different from a conventional method, for example, genomic DNA could be recovered simply without using a special agent. Further, it was confirmed that the concentration of genomic DNA in each of the nucleic acid samples recovered was more than 3000 copies/μL, which was a requisite amount for PCR and the like. Further, a sufficient amount of mitochondrial DNA could also be recovered.

Example 3

First, 50 μL of whole blood and 50 μL of the treatment reagent 1-1 of Example 1 were mixed to prepare 100 μL of a treatment liquid. As a carrier, each of the carriers (Examples 3a to 3g) shown in the following table 4 was cut into a circle having a diameter of 2 mm. Each of the carriers that have been cut and 100 μL of the treatment liquid were put in a tube having a capacity of 1.5 mL and mixed by vortex, and then the treatment liquid was removed. In the tube, 100 μL of the washing liquid of Example 1 was added and mixed by the vortex, and then the washing liquid was discarded. Then, each of the carriers was taken out from the tube, was put in a new tube with 100 μL of the dispersion medium of Example 1, and heated at 96°C for 5 minutes. The dispersion medium after heating was recovered and was used as a nucleic acid sample.

Table 4

| Example | Polymer | Material | Properties | Number of cells in 50 μL of whole blood (number of pieces) | DNA concentration (copy/μL) |
|---|---|---|---|---|---|
| 3a | Polyvinyl chloride | Porous body | Average pore size: 100 μm Thickness: 2 mm Porosity: 62-65% | About 280,000 | 2520 (recovery rate: 45%) |

(continued)

| Example | Polymer | Material | Properties | Number of cells in 50 μL of whole blood (number of pieces) | DNA concentration (copy/μL) |
|---|---|---|---|---|---|
| 3b | PE | Porous body | Average pore size: 150 μm Thickness: 2 mm Porosity: 80% | About 280,000 | 1950 (recovery rate: 35%) |
| 3c | Teflon® | Woven fabric (Mesh type) | Thickness: 290 μm Mesh size: 250 μm Thread diameter: 152 μm | About 280,000 | 1630 (recovery rate: 29%) |
| 3d | Polyester | Woven fabric (Mesh type) | Thickness: 125 μm Mesh size: 100 μm | About 295,000 | 4430 (recovery rate: 75%) |
| 3e | Nylon | Woven fabric (Mesh type) | Thickness: 95 μm Mesh size: 150 μm | About 295,000 | 4360 (recovery rate: 74%) |
| 3f | Polyurethane (Polyether type) | Porous body (Sponge) | Thickness: 2 μm Density: $75\pm10$ kg/m$^3$ | About 350,000 | 4000 (Recovery rate: 68%) |
| 3g | PP | Nonwoven fabric | Thickness: 170 μm Thread diameter: about 2 μm Basis weight: 20 g/m$^3$ | About 350,000 | 3000 (Recovery rate: 51%) |

3a: LV (product name), produced by CELLPOLE INDUSTRIES,CO.,LTD
3b: MAPS (product name), produced by INOAC CORPORATION
3c: PET250-HD (product name), produced by Sefar AG
3d: PET150-HD (product name), produced by SefarAG
3e: 9P-150 (product name), produced by Sefar AG
3f: CFS (product name), produced by INOAC CORPORATION
3g: EM02010 (product name), produced by Toray Fine Chemicals Co., Ltd.
PE: polyethylene
PP: polypropylene

With respect to each of the nucleic acid samples recovered, the concentration of genomic DNA was measured in the same manner as in Example 1, and the recovery rate of the genomic DNA by each of the carriers was calculated. The results thereof are summarized in table 4.

As summarized in table 4, according to the method of Example 3, different from a conventional method, for example, genomic DNA could be recovered simply without using a special agent. Further, it was confirmed that the concentration of genomic DNA in each of the nucleic acid samples recovered was more than 1500 copies/μL,which was a requisite amount for PCR and the like.

Example 4

In a tube having a capacity of 1.5 mL, 50 μL of whole blood and 50 μL of the treatment reagent 1-1 of Example 1 were mixed to prepare 100 $\mu$L of a treatment liquid. Further, as a carrier, a total amount of 1mg of each of the magnetic beads (Examples 4a and 4b) in the following table 5 was added to the tube and mixed by vortex. The magnetic beads were recovered by means of a magnet and the treatment liquid in the tube was removed. Then, the magnetic beads recovered were put in the tube with 100 $\mu$L of the washing liquid of Example 1 and mixed by vortex, and the magnetic beads were recovered again and the washing liquid was removed again. Then, in the tube, the magnetic beads recovered and 100 $\mu$L of the dispersion medium of Example 1 were added and mixed by vortex, and heated at 96°C for 5 minutes. After heating, the tube was subjected to centrifugal separation to remove the magnetic beads, and the dispersion medium was recovered. This was used as a nucleic acid sample.

Table 5

| Example | Magnetic bead | Properties | Number of cells in 50 $\mu$L of whole blood (number of pieces) | DNA concentration (copy/$\mu$L) |
|---|---|---|---|---|
| 4a | PS+maleic acid (PEG-COOH) | Particle size: 5$\mu$m Total superficial area: 11cm$^2$ | About 250,000 | 495 |
| 4b | PS+maleic acid (Alkyl-OH) | Pore size: 5$\mu$m Total superficial area: 11cm$^2$ | About 250,000 | 970 |
| 4a and 4b: micromer® M (product name), produced by micromod PS: polystyrene | | | | |

With respect to each of the nucleic acid samples recovered, the concentration of genomic DNA was measured in the same manner as in Example 1, and the recovery rate of the genomic DNA by each of the carriers was calculated. The results thereof are summarized in table 5.

As summarized in table 5, according to the method of Example 4, different from a conventional method, for example, DNA could be recovered simply without using a special agent. Further, it was confirmed that the concentration of DNA in each of the nucleic acid samples recovered was more than 400 copies/$\mu$L, which was a requisite amount for PCR and the like.

Reference Example 1

Whole blood was treated with the nucleic acid recovery reagent (treatment reagent) of the present invention, and nucleic acid complexes held in a filter-shaped carrier were observed.

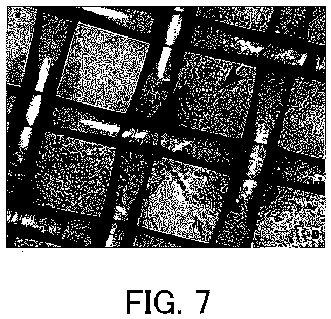

10 $\mu$L of whole blood and 10 $\mu$L of the treatment reagent in the following table 6 were mixed to prepare 20 $\mu$L of a treatment liquid. As a carrier, a filter (nylon mesh (product name), produced by Sefar AG) having a mesh opening size of 100 $\mu$m was used. 20 $\mu$L of the treatment liquid was passed through the carrier in the same manner as in Example 1. Then, the carrier was subjected to Giemsa staining according to a known method and was observed with a light microscope. The result thereof is shown in FIG. 7. In the light micrograph of FIG. 7, as indicated by the arrow, it was observed that a nucleic acid complex having a diameter of about 200 $\mu$m stained purple was attached to the carrier.

Table 6

(Treatment reagent)

| | |
|---|---|
| Proteinase K | 1 $\mu$g (27mU)/$\mu$L |
| Tris-HCl (pH 8.9) | 20 mmol/L |
| Nonidet® P-40 | 0.5% |
| $CaCl_2$ | 1 mol/L |

Example 5

Recovery of RNA from Whole Blood

As a carrier, MF-80A (product name, produced by INOAC CORPORATION) was used. This carrier was cut into a circle having a diameter of 2 mm and attached to a jig in the same manner as in Example 1. Three kinds of whole blood (fresh blood) collected from different specimens were provided, and 50 μL of each of the whole blood and 50 μL of the treatment reagent 5-1 in the following table 7 were mixed to prepare 100 μL of a treatment liquid. In the treatment liquid, relative to 50 μL of the whole blood, the amount of the protease was 1.35 U and the amount of the surfactant was 0.25 vol%. Then, nucleic acid samples were prepared in the same manner as in Example 1 except that the washing liquid and the dispersion medium in the following table 7 were used. With respect to each of the nucleic acid samples recovered, a quantitative analysis of RNA was performed using One Step SYBR Prime Script RT-PCR Kit II (TAKARA) and the following primer set for β-actin. As a control, with respect to each of the nucleic acid samples purified from the same whole blood as the aforementioned three kinds of whole blood using QIAamp RNA Blood Mini (produced by QIAGEN), a quantitative analysis of RNA was performed in the same manner as described above. Then, the relative value (%) of the amount of each of the RNAs recovered in Example 5 was obtained assuming that the amount of each of the RNAs recovered in the control is 100%.

Table 7

| | |
|---|---|
| (Treatment reagent 5-1) | |
| Proteinase K | 1 μg (27mU)/μL |
| Tris-HCl (pH 8.6) | 20 mmol/L |
| Nonidet® P-40 | 0.5% |
| CaCl$_2$ | 1 mmol/L |
| (Washing liquid and Dispersion medium) | |
| RNase Inhibitor | 200 mU/μL |
| (product name: SUPERase·InTMRNase Inhibitor, produced by Ambion, Inc.) | |
| RNase free H$_2$O | |
| (Primer set for β-actin) | |
| Forward primer: TGAACCCCAAGGCCAACCGC (SEQ ID NO:1) | |
| Reverse primer: TTGTGCTGGGTGCCAGGGCA (SEQ ID NO:2) | |

The results of the three specimens were 5.6%, 12.3%, and 7.2%. These recovery rates are sufficient amounts as templates for RT-PCR, for example. Therefore, according to Example 5, different from a conventional method, RNA of a requisite amount could be recovered simply without using a special agent.

Example 6

Recovery of Genomic DNA from Saliva

100 μL of saliva (the number of human nucleic acid-containing cells: about 150,000) and 100 μL of each of the treatment reagents in the following table 8 were mixed to prepare 200 μL of a treatment liquid. In the treatment liquids, relative to 50 μL of the saliva, the amount of protease was 1 U and the amounts of the surfactants were 0.5 vol%, 1 vol%, and 2 vol%. As a carrier, mesh type woven fabric made of PET having a pore size of 60 μm (produced by SANPLATEC CO.,LTD., mesh size: 60 μm) was used. This carrier was cut into a circle having a diameter of 3.5 mm and attached to the inside of the pipette chip having a capacity of 200 μL shown in FIG. 1 (effective diameter: 2.2 mm). The effective diameter denotes a diameter of the carrier attached to the inside of the pipette chip through which a liquid can actually be passed. This pipette chip was attached to a pipetter, 200 μL of each of the treatment liquids was sucked into the pipette chip and drained from the pipette chip, and then 200 μL of the washing liquid of Example 1 was sucked into the pipette chip and drained from the pipette chip. Once again, 200 μL of the unused washing liquid was sucked into the pipette chip and drained from the pipette chip, and then the carrier was taken out from the pipette chip. This carrier was immersed in 100 μL of the dispersion medium of Example 1 and heated at 95°C for 5 minutes to obtain a nucleic acid sample. With respect to each of the nucleic acid samples, the concentration of genomic DNA was measured in the same manner as in Example 1. The results thereof are summarized in the following table 8.

Table 8

| | Treatment reagent 6-1 | Treatment reagent 6-2 | Treatment reagent 6-3 |
|---|---|---|---|
| Proteinase K | 40 mU/μL | 40 mU/μL | 40 mU/μL |
| Tris-HCl (pH8.6) | 20 mmol/L | 20 mmol/L | 20 mmol/L |
| Nonidet® P-40 | 1% | 2% | 4% |
| CaCl$_2$ | 1 mmol/L | 1 mmol/L | 1 mmol/L |

(continued)

|  | Treatment reagent 6-1 | Treatment reagent 6-2 | Treatment reagent 6-3 |
|---|---|---|---|
| Genomic DNA concentration (copy/$\mu$L) | 3,160 | 4,700 | 2,650 |
| %: v/v% (hereinafter, the same applies) | | | |

As summarized in table 8, according to the method of Example 6, also with respect to the saliva, genomic DNA having a concentration of about 3000 copies/$\mu$L, which was a sufficient amount as a template for PCR, could be recovered.

Example 7

Recovery of Genomic DNA from Oral Mucosa Cell

The right and left sides of the inside of a subject's cheek were rubbed with a cotton swab (1P1501V, produced by JCB Industry Limited) in a circular motion 10 times for each. The cotton swab was added to 200 $\mu$L of each of the treatment reagents in the following table 9 to prepare 200 $\mu$L of a treatment liquid (the number of human nucleic acid-containing cells: about 250,000). In the treatment liquids, relative to 50 $\mu$L of the oral mucosa cell suspension, the amount of protease was 1 U and the amounts of the surfactants were 0.5 vol%, 1 vol%, and 2 vol%. Using the pipette chip to which the carrier of Example 6 was attached, nucleic acid samples were prepared in the same manner as in Example 6. With respect to each of the nucleic acid samples, the concentration of genomic DNA was measured in the same manner as in Example 1. The results thereof are summarized in the following table 9.

Table 9

|  | Treatment reagent 7-1 | Treatment reagent 7-2 | Treatment reagent 7-3 |
|---|---|---|---|
| Proteinase K | 20 mU/$\mu$L | 20 mU/$\mu$L | 20 mU/$\mu$L |
| Tris-HCl (pH8.6) | 10 mmol/L | 10 mmol/L | 10 mmol/L |
| Nonidet® P-40 | 0.25% | 0.5% | 1% |
| CaCl$_2$ | 0.5 mmol/L | 0.5 mmol/L | 0.5 mmol/L |
| Genomic DNA concentration (copy/$\mu$L) | 963 | 1,890 | 2,390 |

As summarized in table 9, according to the method of Example 7, also with respect to the oral mucosa cell, genomic DNA having a concentration of about 1000 to 2000 copies/$\mu$L, which was a sufficient amount as a template for PCR, could be recovered.

Example 8

Recovery of Genomic DNA from Cultured Cell

A suspension of Asian-derived normal human umbilical vein endothelial cells (KJB-110, DS PHARMA BIOMEDICAL) that had been cultured was washed with saline to remove culture medium, and the cultured cells were suspended in the saline so as to be of $1 \times 10^3$ cells/$\mu$L. 100 $\mu$L of the suspension (the number of nucleic acid-containing cells: about $1 \times 10^5$) was mixed with 100 $\mu$L of each of the treatment reagents in the following table 10 to prepare 200 $\mu$L of a treatment liquid. In the treatment liquids, relative to 50 $\mu$L of the suspension, the amount of protease was 1 U and the amounts of the surfactants were 0.5 vol%, 1 vol%, and 2 vol%. Using the pipette chip to which the carrier of Example 6 was attached, nucleic acid samples were prepared in the same manner as in Example 6. With respect to each of the nucleic acid samples, the concentration of genomic DNA was measured in the same manner as in Example 1. The results thereof are summarized in the following table 10.

Table 10

|  | Treatment reagent 8-1 | Treatment reagent 8-2 | Treatment reagent 8-3 |
|---|---|---|---|
| Proteinase K | 40 mU/$\mu$L | 40 mU/$\mu$L | 40 mU/$\mu$L |
| Tris-HCl (pH8.6) | 20 mmol/L | 20 mmol/L | 20 mmol/L |
| Nonidet® P-40 | 1% | 2% | 4% |

(continued)

|  | Treatment reagent 8-1 | Treatment reagent 8-2 | Treatment reagent 8-3 |
|---|---|---|---|
| CaCl$_2$ | 1 mmol/L | 1 mmol/L | 1 mmol/L |
| Genomic DNA concentration (copy/μL) | 153 | 588 | 852 |

As summarized in table 10, according to the method of Example 8, also with respect to the cultured cells, genomic DNA having a concentration of about hundreds copies/μL, which was a sufficient amount as a template for PCR, could be recovered.

Example 9

Recovery of Plasmid DNA from Escherichia Coli

After culturing Escherichia coli having plasmid DNA, to which an IAPP (islet amyloid polypeptide) sequence was integrated, in an LB culture medium for 12 hours, 100 μL of the cultured solution (the number of nucleic acid-containing cells: about $1\times10^7$) was mixed with 100 μL of each of the following treatment reagents to prepare 200 μL of a treatment liquid. With respect to Escherichia coli to be treated with the treatment reagent 9-1 in the following table 11, after cultivation, the cultured solution was directly frozen at -80°C and thawed at 25°C at the time of using. Thereafter, the cultured solution was mixed with the treatment reagent 9-1 in the same manner as described above. In the treatment liquids, relative to 50 μL of the cultured cell suspension, the amounts of protease were 0 U and 1 U and the amounts of the surfactants were 0 vol%, 0.5 vol%, 1 vol%, and 2 vol%. Using the pipette chip to which the carrier of Example 6 was attached, nucleic acid samples were prepared in the same manner as in Example 6. With respect to each of the nucleic acid samples, the concentration of plasmid DNA was measured in the same manner as in Example 1. Further, with respect to each of the nucleic acid samples, the concentration of genomic DNA was measured by an absorbance measurement at the wavelength of 260 nm. The results thereof are summarized in the following table 11.

Table 11

|  | Treatment reagent 9-1 | Treatment reagent 9-2 | Treatment reagent 9-3 | Treatment reagent 9-4 |
|---|---|---|---|---|
| Proteinase K<br>Tris-HCl (pH8.6)<br>Nonidet® P-40 | -<br>20 mmol/L<br>- | 40 mU/μL<br>20 mmol/L<br>1% | 40 mU/μL<br>20 mmol/L<br>2% | 40 mU/μL<br>20 mmol/L<br>4% |
| Plasmid DNA concentration (copy/μL) | 528,096 | 585,501 | 951,689 | 248,783 |
| Genomic DNA concentration (copy/μL) | 31,766 | 31,202 | 35,681 | 33,021 |

As summarized in table 11, according to the method of Example 9, also with respect to the cultured cell of Escherichia coli, plasmid DNA having a concentration of about 500,000 copies/μL, which was a sufficient amount as a template for PCR, and genomic DNA having a concentration of about 30,000 copies/μL, which was a sufficient amount as a template for PCR, could be recovered. Further, from the result of the case in which Escherichia coli was treated with the treatment reagent 9-1, it was found that, by freeze-thawing the cultured cells of Escherichia coli, plasmid DNA and genomic DNA comparable to those described above could be recovered without using protease and a surfactant.

Example 10

Recovery of Genomic DNA from Whole Blood

100 μL of whole blood that had been refrigerated at 4°C for 70 days and 100 μL of whole blood that has been frozen at -30°C for 9 months and then thawed were each mixed with 100 μL of each of the treatment reagents in the following table 12 to prepare 200 μL of a treatment liquid. The number of nucleic acid-containing cells in 100 μL of the refrigerated whole blood was about 580,000 and the number of nucleic acid-containing cells in 100 μL of the freeze-thawed whole blood was about 560,000. In the treatment liquids, relative to 50 μL of the whole blood, the amounts of protease were 1U and 2 U and the surfactant was not added. Using the pipette chip to which the carrier of Example 6 was attached, nucleic acid samples were prepared in the same manner as in Example 6. With respect to each of the nucleic acid samples, the concentration of genomic DNA was measured in the same manner as in Example 1. The results thereof are summarized in the following table 12.

Table 12

|  | Treatment reagent 10-1 | Treatment reagent 10-2 |
|---|---|---|
| Proteinase K<br>Tris-HCl (pH8.6)<br>$CaCl_2$ | 40 mU/$\mu$L<br>20 mmol/L<br>1 mmol/L | 20 mU/$\mu$L<br>20 mmol/L<br>1 mmol/L |
| Refrigerated whole blood Genomic DNA concentration (copy/$\mu$L) | 10,500 | 6,930 |
| Frozen whole blood Genomic DNA concentration (copy/$\mu$L) | 9,080 | 11,200 |

As summarized in table 12, according to the method of Example 10, by refrigerating or freeze-thawing the whole blood, genomic DNA of a sufficient amount as a template for PCR could be recovered without using a surfactant.

Example 11

1. Recovery of Genomic DNA from Fresh Blood

Fresh whole blood on the day of collection, which has not been refrigerated or frozen was treated under various conditions as described below, and then genomic DNA was recovered. The number of leukocytes in the fresh whole blood was about 5,500/$\mu$L.

In a tube, 50 $\mu$L of the treatment reagent 6 in the following table 13 and 50 $\mu$L of fresh whole blood were added to prepare a treatment liquid. The concentrations of protease and a surfactant in the treatment reagent 6 are shown in the following table 14. The concentrations of protease and a surfactant in the treatment liquid are half of the concentrations shown in table 14 (hereinafter, the same applies). As a carrier, mesh type woven fabric made of PET having a pore size of 105 $\mu$m (PET105 (product name), produced by Sefar AG, mesh size: 105 $\mu$m) was provided. This carrier was cut into a circle having a diameter of 2.5 mm and attached to the inside of the pipette chip having a capacity of 200 $\mu$L shown in FIG. 1 (effective diameter: 1.4 mm). This pipette chip was attached to a pipetter, an operation of sucking 100 $\mu$L of each of the treatment liquids into the pipette chip and draining from the pipette chip was performed 20 times, and then the treatment liquid was drained. Subsequently, in a new tube, 100 $\mu$L of a washing liquid 1 (250 mmol/L KCL solution) was added, an operation of sucking 100 $\mu$L of the washing liquid 1 into the pipette chip and draining from the pipette chip was performed 10 times, and then the washing liquid 1 was drained. Further, in a new tube, 100 $\mu$L of a washing liquid 2 (0.1 mmol/L EDTA and 1 mmol/L Tris-HCl (pH: 8.0)) was added, an operation of sucking 100 $\mu$L of the washing liquid 2 into the pipette chip and draining from the pipette chip was performed 10 times, and then the washing liquid 2 was drained. Thereafter, the carrier was taken out from the pipette chip. This carrier was immersed in 100 $\mu$L of a dispersion medium (0.1 mmol/L EDTA and 1mmol/L Tris-HCl (pH: 8.0)) and was heated in that condition at 95°C for 5 minutes, and thereby a nucleic acid sample was obtained. With respect to each of the nucleic acid samples, the concentration of genomic DNA was measured in the same manner as in Example 1. The results thereof are summarized in the following table 14.

It is to be noted that examples in which a treatment reagent that contains protease and a surfactant is used are referred to as Examples A1 to A6 and examples in which a treatment reagent that contains one of protease and a surfactant or a treatment reagent that contains neither protease nor a surfactant is used are referred to as Comparative Examples A1 to A3.

Table 13

(Treatment reagent 6)

| Proteinase K | Predetermined concentration |
|---|---|
| Nonidet® P-40 | Predetermined concentration |
| Tris-HCl (pH 8.0) | 10 mmol/L |

Table 14

| | Protease concentration* | Surfactant concentration* | DNA concentration (copy/$\mu$L) |
|---|---|---|---|
| Example A1 | 2 U/$\mu$L | 1 vol% | 3780 |
| Example A2 | 20 mU/$\mu$L | 1 vol% | 3540 |
| Example A3 | 4 mU/$\mu$L | 1 vol% | 318 |
| Example A4 | 20 mU/$\mu$L | 20 vol% | 113 |
| Example A5 | 20 mU/$\mu$L | 0.6 vol% | 1630 |
| Example A6 | 20 mU/$\mu$L | 0.3 vol% | 323 |
| Comparative Example A1 | - | - | 0 |
| Comparative Example A2 | 20 mU/$\mu$L | - | 0 |
| Comparative Example A3 | - | 1 vol% | 1 |
| * concentration in treatment liquid 6 (hereinafter, the same applies) | | | |

As summarized in table 14, according to the method of Example 11, genomic DNA having a concentration of about 100 copies/$\mu$L, which was a sufficient amount as a template for PCR, could be recovered from the fresh whole blood. In contrast, with respect to Comparative Example A1 in which a treatment reagent that contains neither protease nor a surfactant is used and Comparative Examples A2 and A3 in which a treatment reagent that does not contain one of protease and a surfactant is used, very little DNA was recovered.

2. Recovery of Genomic DNA from Refrigerated Blood

Whole blood that had been refrigerated for 2 months was treated under various conditions as described below, and then genomic DNA was recovered. The number of leukocytes in the refrigerated blood was about 6,900/$\mu$L.
The recovery of genomic DNA and the measurement thereof were performed in the same manner as the fresh whole blood except that the concentrations of the protease and the surfactant in the treatment reagent 6 were the concentrations shown in the following table 15.

Table 15

| | Protease concentration* | Surfactant concentration* | DNA concentration (copy/$\mu$L) |
|---|---|---|---|
| Example B1 | 20 mU/$\mu$L | - | 1450 |
| Example B2 | 20 mU/$\mu$L | 1 vol% | 6650 |
| Comparative Example B1 | - | - | 12 |

As shown by the result of Example B1 summarized in table 15, when blood after refrigeration was used, by heat-treating the refrigerated blood as described above after treating with a treatment reagent that contains protease but does not contain a surfactant, genomic DNA of a sufficient amount as a template for PCR could be recovered. In this manner, it was found that, in the case where the whole blood was refrigerated, DNA could be recovered efficiently even when the treatment reagent does not contain the surfactant. Further, as shown by the result of the Example B2 summarized in table 15, it was found that the recovery rate of genomic DNA could further be increased when the treatment reagent further contains a surfactant besides protease. In contrast, as summarized in table 15, with respect to Comparative Example B1 in which a treatment reagent that contains neither protease nor a surfactant is used, very little DNA was recovered.

3. Recovery of Genomic DNA from Frozen Blood

Whole blood that had been frozen at -30°C for 2 hours was thawed at room temperature and treated under various conditions as described below, and then genomic DNA was recovered. The number of leukocytes in the frozen blood was about 5,500/$\mu$L.
The recovery of genomic DNA and the measurement thereof were performed in the same manner as the fresh

**EP 2 383 335 B1**

whole blood except that the concentrations of the protease and the surfactant in the treatment reagent 6 were the concentrations shown in the following table 16.

Table 16

|  | Protease concentration* | Surfactant concentration* | DNA concentration (copy/μL) |
|---|---|---|---|
| Example C1 | - | - | 617 |
| Example C2 | 0.2 mU/μL | - | 739 |
| Example C3 | 20 mU/μL | - | 3230 |
| Example C4 | 20 mU/μL | 1 vol% | 5020 |

As shown by the result of the Example C1 summarized in table 16, when blood after freeze-thawing was used, by heat-treating the blood in the same manner as described above, genomic DNA of a sufficient amount as a template for PCR could be recovered even when a treatment reagent contains neither protease nor a surfactant. As shown by the results of the Examples C2 and C3, by using treatment reagents that contain the protease, the recovery rate of DNA could be increased. Further, as shown by the result of the Example C4, by using a treatment reagent that contains protease and a surfactant, the recovery rate of DNA could further be increased.

Industrial Applicability

As described above, according to the present invention, nucleic acid can be recovered simply without using a special reagent, for example. Further, according to the present invention, nucleic acid can be recovered efficiently from cells. For example, besides genomic DNA and RNA, mitochondrial DNA also can be recovered. In this manner, since the present invention is superior in the recovery rate of nucleic acid as well as in safety and operability, for example, a sufficient amount of template nucleic acid required for nucleic acid amplification or the like can be recovered from a small amount of cell sample. Further, since the recovery of nucleic acid from a small amount of cell sample can be performed by a simple operation, for example, it may be said that the present invention is useful for nucleic acid amplification and nucleic acid recovery using a microchip, micro-TAS, or the like, which have received attention recently.

**Claims**

1. A method for producing a nucleic acid sample by recovering nucleic acid from cells, comprising the steps of

    (A) with respect to a cell sample containing cells, releasing nucleic acid complexes from the cells, wherein the cell sample is mixed with a treatment reagent containing a protease and a surfactant in an aqueous solvent to generate a treatment liquid and wherein said nucleic acid complexes have a diameter of 50 to 200$\mu$m;
    (B) contacting the treatment liquid containing the cell sample from step (A) with a carrier in a container, wherein said carrier is capable of capturing said released nucleic acid complexes and the carrier is at least one selected from the group consisting of nonwoven fabric, woven fabric, porous bodies and a bead ;
    (C) separating the carrier from the treatment liquid, wherein the carrier is separated from the treatment liquid by taking the carrier or the treatment liquid out of the container;
    (D) applying heat treatment to the carrier at 90°C to 100 °C for 1 minute to 5 minutes; and
    (E) adding a dispersion medium which is an aqueous solvent to the carrier before or after step (D),

    wherein a nucleic acid-insoluble organic solvent is not used in said method.

2. The method according to claim 1, wherein
the concentration of the protease is 0.5 mU/$\mu$L or more and the concentration of the surfactant is 0.1 vol% or more in the treatment liquid obtained by mixing the treatment reagent and the cell sample.

3. The method according to claim 2 wherein, in the treatment liquid, the concentration of the protease is 0.5 mU/$\mu$L to 1000 mU/$\mu$L and the concentration of the surfactant is 0.1 vol% to 20 vol%.

4. The method according to any one of claims 1 to 3, wherein, in the treatment liquid, the amount of the protease relative to $1\times10^2$ to $1\times10^9$ cells is 0.02 mU or more, and the amount of the surfactant relative to $1\times10^2$ to $1\times10^9$ cells is 1 femtomole ($1\times10^{-15}$ mole) or more, and/or wherein, in the treatment liquid, the amount of the protease relative to 50 $\mu$L of the cell sample is 0.02 mU or more and the amount of the surfactant relative to 50 $\mu$L of the cell sample is 1 femtomole or more.

5. The method according to any one of claims 1 to 4, wherein the protease is at least one selected from the group consisting of proteinase K, chymotrypsin, pepsin, cathepsin D, and papain, and/or wherein the surfactant is a nonionic surfactant.

6. The method according to claim 5 wherein the nonionic surfactant is at least one selected from the group consisting of polyoxyethylene-p-isooctylphenol, polyoxyethylene sorbitan monolaurate, polyoxyethylene-nonylphenyl ether, and nonylphenol polythioethoxylate.

7. The method according to any one of claims 1 to 6, wherein the treatment reagent contains at least one of a chelating agent and a protein denaturing agent.

8. The method according to any one of claims 1 to 7, wherein the cells are at least one selected from the group consisting of blood cells, cells in saliva, oral mucosa cells, somatic cells, germ cells, and tumor cells or wherein the cells are cultured cells.

9. The method according to any one of claims 1 to 8, wherein the cell sample is at least one selected from the group consisting of blood, saliva, oral mucosa, nails, and hairs, and/or wherein the cell sample is a culture of the cells, and/or the cell sample is an undiluted cell sample.

10. The method according to any one of claims 1 to 9, wherein the cells are prokaryotic cells, and step (A) is a step of freeze-thawing or refrigerating a cell sample containing the prokaryotic cells.

11. The method according to any one of claims 1 to 10, wherein the carrier is a porous body having an average pore size of 10 $\mu$m to 1000 $\mu$m, or wherein the carrier is a nonwoven fabric having a basis weight of 20 g/m$^3$ to 150 g/m$^3$, or wherein the carrier is a woven fabric having a mesh size of 10 $\mu$m to 1000 $\mu$m.

12. The method according to any one of claims 1 to 11, wherein the shape of the carrier is at least one selected from the group consisting of a filter, a sheet, a block, and a bead.

13. The method according to claim 12 wherein the carrier is a bead having a volume filling fraction of 0.1% to 10%.

14. The method according to any one of claims 1 to 13, wherein the carrier is at least one of a polymer carrier and a metal carrier.

15. The method according to claim 14 wherein the polymer is at least one selected from the group consisting of polypropylene, polyethylene, polyester, polyamide, polyurethane, polyether, polystyrene, polyvinyl chloride, and polytetrafluoroethylene, and/or wherein the metal is at least one selected from the group consisting of stainless steel, titanium, and aluminum.

16. The method according to any one of claims 1 to 15, wherein the nucleic acid is at least one of DNA and RNA.

17. The method according to claim 16 wherein the DNA is at least one of genomic DNA and mitochondrial DNA.

18. A method for producing a nucleic acid amplification product having a target sequence by a nucleic acid amplification method, comprising the steps of

(a) recovering a nucleic acid sample from a cell sample containing cells by the method for producing a nucleic acid sample according to any one of claims 1 to 17; and
(b) producing, using nucleic acid in the nucleic acid sample recovered in step (a) as a template, a nucleic acid amplification product having a target sequence in the template by a nucleic acid amplification method.

**Patentansprüche**

1. Verfahren zur Herstellung einer Nukleinsäureprobe durch Gewinnung von Nukleinsäure aus Zellen, umfassend die Schritte des:

(A) in Bezug auf eine Zellen enthaltende Zellprobe, Freisetzens von Nukleinsäurekomplexen aus den Zellen, wobei die Zellprobe mit einem Behandlungsreagens gemischt wird, das eine Protease und ein oberflächenaktives Mittel in einem wässrigen Lösungsmittel enthält, um eine Behandlungsflüssigkeit zu erzeugen, und wobei die Nukleinsäurekomplexe einen Durchmesser von 50 bis 200 $\mu$m aufweisen;
(B) Inkontaktbringens der Behandlungsflüssigkeit, welche die Zellprobe von Schritt (A) enthält, mit einem Träger in einem Behälter, wobei der Träger fähig ist, die freigesetzten Nukleinsäurekomplexe zu binden und der Träger mindestens eines ist, das aus der aus Vliesstoff, Gewebe, porösen Körpern und einem Kügelchen bestehenden Gruppe ausgewählt ist;
(C) Abscheidens des Trägers von der Behandlungsflüssigkeit, wobei der Träger von der Behandlungsflüssigkeit abgeschieden wird, indem der Träger oder die Behandlungsflüssigkeit aus dem Behälter genommen wird;
(D) Anwendens von Wärmebehandlung auf den Träger bei 90 °C bis 100 °C für einen Zeitraum von 1 Minute bis 5 Minuten; und
(E) Zusetzens eines Dispersionsmediums, das ein wässriges Lösungsmittel ist, zu dem Träger, vor oder nach Schritt (D),

wobei in diesem Verfahren kein nukleinsäureunlösliches organisches Lösungsmittel verwendet wird.

2. Verfahren nach Anspruch 1, wobei in der durch Mischen des Behandlungsreagens und der Zellprobe erhaltenen Behandlungsflüssigkeit die Konzentration der Protease 0,5 mU/$\mu$l oder mehr beträgt und die Konzentration des oberflächenaktiven Mittels 0,1 Vol% oder mehr beträgt.

3. Verfahren nach Anspruch 2, wobei, in der Behandlungsflüssigkeit, die Konzentration der Protease 0,5 mU/$\mu$l bis 1000 mU/$\mu$l beträgt und die Konzentration des oberflächenaktiven Mittels 0,1 Vol% bis 20 Vol% beträgt.

4. Verfahren nach einem der Ansprüche 1 bis 3, wobei, in der Behandlungsflüssigkeit, die Menge an Protease in Bezug auf 1x10$^2$ bis $1\times10^9$ Zellen 0,02 mU oder mehr beträgt und die Menge an oberflächenaktivem Mittel in Bezug auf 1x10$^2$ bis 1x10$^9$ Zellen 1 Femtomol (1x10$^{-15}$ mol) oder mehr beträgt, und/oder wobei, in der Behandlungsflüssigkeit, die Menge an Protease in Bezug auf 50 $\mu$l der Zellprobe 0,02 mU oder mehr beträgt und die Menge an oberflächenaktivem Mittel in Bezug auf 50 $\mu$l der Zellprobe 1 Femtomol oder mehr beträgt.

5. Verfahren nach einem der Ansprüche 1 bis 4, wobei die Protease mindestens eines ist, ausgewählt aus der aus Proteinase K, Chymotrypsin, Pepsin, Cathepsin D und Papain bestehenden Gruppe, und/oder wobei das oberflächenaktive Mittel ein nichtionisches oberflächenaktives Mittel ist.

6. Verfahren nach Anspruch 5, wobei das nichtionische oberflächenaktive Mittel mindestens eines ist, ausgewählt aus der aus Polyoxyethylene-p-isooctylphenol, Polyoxyethylensorbitanmonolaurat, Polyoxyethylen-nonylphenylether und Nonylphenolpolythioethoxylat bestehenden Gruppe.

7. Verfahren nach einem der Ansprüche 1 bis 6, wobei das Behandlungsreagens aus mindestens einem eines Chelatbildners und eines Proteindenaturierungsmittels besteht.

8. Verfahren nach einem der Ansprüche 1 bis 7, wobei die Zellen mindestens eines sind, ausgewählt aus der aus Blutzellen, Zellen in Speichel, Mundschleimhautzellen, somatischen Zellen, Keimzellen und Tumorzellen bestehenden Gruppe, oder wobei die Zellen kultivierte Zellen sind.

9. Verfahren nach einem der Ansprüche 1 bis 8, wobei die Zellprobe mindestens eines ist, ausgewählt aus der aus Blut, Speichel, Mundschleimhaut, Nägeln und Haaren bestehenden Gruppe, und/oder wobei die Zellprobe eine Kultur der Zellen ist und/oder die Zellprobe eine unverdünnte Zellprobe ist.

10. Verfahren nach einem der Ansprüche 1 bis 9, wobei die Zellen prokaryotische Zellen sind und Schritt (A) ein Schritt des Einfrierens-Auftauens oder Kühlens einer die prokaryotischen Zellen enthaltenden Zellprobe ist.

11. Verfahren nach einem der Ansprüche 1 bis 10, wobei der Träger ein poröser Körper mit einer durchschnittlichen Porengröße von 10 $\mu$m bis 1000 $\mu$m ist, oder wobei der Träger ein Vlies mit einem Basisgewicht von 20 g/m$^3$ bis 150 g/m$^3$ ist, oder wobei der Träger ein Gewebe mit einer Maschengröße von 10 $\mu$m bis 1000 $\mu$m ist.

12. Verfahren nach einem der Ansprüche 1 bis 11, wobei die Form des Trägers mindestens eine ist, ausgewählt aus der aus einem Filter, einem Bogen, einem Block und einem Kügelchen bestehenden Gruppe.

13. Verfahren nach Anspruch 12, wobei der Träger ein Kügelchen mit einem Volumenfüllanteil von 0,1 % bis 10 % ist.

14. Verfahren nach einem der Ansprüche 1 bis 13, wobei der Träger mindestens eines eines Polymerträgers und eines Metallträgers ist.

15. Verfahren nach Anspruch 14, wobei das Polymer mindestens eines ist, ausgewählt aus der aus Polypropylen, Polyethylen, Polyester, Polyamid, Polyurethan, Polyether, Polystyrol, Polyvinylchlorid und Polytetrafluorethylen bestehenden Gruppe, und/oder wobei das Metall mindestens eines ist, ausgewählt aus der aus Edelstahl, Titan und Aluminium bestehenden Gruppe.

16. Verfahren nach einem der Ansprüche 1 bis 15, wobei die Nukleinsäure mindestens eines von DNA und RNA ist.

17. Verfahren nach Anspruch 16, wobei die DNA mindestens eines von genomischer DNA und mitochondrialer DNA ist.

18. Verfahren zur Herstellung eines Nukleinsäureamplifikationsprodukts mit einer Zielsequenz durch ein Nukleinsäureamplifikationsverfahren, umfassend die Schritte des:

(a) Gewinnens einer Nukleinsäureprobe aus einer Zellprobe, die Zellen enthält, mittels des Verfahrens zur Herstellung einer Nukleinsäureprobe nach einem der Ansprüche 1 bis 17; und

(b) Produzierens, unter Anwendung von Nukleinsäure in der in Schritt (a) gewonnenen Nukleinsäureprobe als Matrize, eines Nukleinsäureamplifikationsprodukts mit einer Zielsequenz in der Matrize, durch ein Nukleinsäureamplifikationsverfahren.

## Revendications

1. Procédé de production d'un échantillon d'acide nucléique par récupération d'acide nucléique à partir de cellules, comprenant les étapes :

(A) en ce qui concerne un échantillon cellulaire contenant des cellules, de libération de complexes d'acide nucléique à partir des cellules, dans lequel l'échantillon cellulaire est mélangé avec un réactif de traitement contenant une protéase et un tensioactif dans un solvant aqueux pour générer un liquide de traitement et dans lequel lesdits complexes d'acide nucléique ont un diamètre de 50 à 200 $\mu$m ;

(B) de mise en contact du liquide de traitement contenant l'échantillon cellulaire issu de l'étape (A) avec un support dans un récipient, dans lequel ledit récipient est capable de capturer lesdits complexes d'acide nucléique libérés et le support est au moins l'un choisi dans le groupe constitué de tissu non tissé, de tissu tissé, de corps poreux et d'une bille ;

(C) de séparation du support du liquide de traitement, dans lequel le support est séparé du liquide de traitement par prélèvement du support ou du liquide de traitement hors du récipient ;

(D) d'application d'un traitement à la chaleur sur le support à 90 °C à 100 °C pendant 1 minute à 5 minutes ; et

(E) d'addition d'un milieu de dispersion qui est un solvant aqueux au support avant ou après l'étape (D),

dans lequel un acide nucléique-solvant organique insoluble n'est pas utilisé dans ledit procédé.

2. Procédé selon la revendication 1, dans lequel
la concentration de la protéase est de 0,5 mU/$\mu$l ou plus et la concentration du tensioactif est de 0,1 % en volume ou plus dans le liquide de traitement obtenu en mélangeant le réactif de traitement et l'échantillon cellulaire.

3. Procédé selon la revendication 2 dans lequel, dans le liquide de traitement, la concentration de la protéase est de 0,5 mU/$\mu$l à 1000 mU/$\mu$l et la concentration du tensioactif est de 0,1 % en volume à 20 % en volume.

4. Procédé selon l'une quelconque des revendications 1 à 3, dans lequel, dans le liquide de traitement, la quantité de la protéase par rapport à 1 x $10^2$ à 1 x $10^9$ cellules est de 0,02 mU ou plus, et la quantité du tensioactif par rapport à 1 x $10^2$ à 1 x $10^9$ cellules est de 1 femtomole (1 x $10^{-15}$ mole) ou plus, et/ou dans lequel, dans le liquide de traitement, la quantité de la protéase par rapport à 50 $\mu$l de l'échantillon cellulaire est de 0,02 mU ou plus et la quantité du tensioactif par rapport à 50 $\mu$l de l'échantillon cellulaire est de 1 femtomole ou plus.

5. Procédé selon l'une quelconque des revendications 1 à 4, dans lequel la protéase est au moins l'une choisie dans le groupe constitué de protéinase K, chymotrypsine, pepsine, cathepsine D, et papaïne, et/ou dans lequel le tensioactif est un tensioactif non ionique.

6. Procédé selon la revendication 5 dans lequel le tensioactif non ionique est au moins l'un choisi dans le groupe constitué de polyoxyéthylène-p-iso-octylphénol, monolaurate de polyoxyéthylène sorbitane, polyoxyéthylène-éther de nonylphényle, et polythioéthoxylate de nonylphénol.

7. Procédé selon l'une quelconque des revendications 1 à 6, dans lequel le réactif de traitement contient au moins un agent chélateur et un agent dénaturant les protéines.

8. Procédé selon l'une quelconque des revendications 1 à 7, dans lequel les cellules sont au moins les unes choisies dans le groupe constitué de cellules sanguines, cellules dans la salive, cellules de muqueuse buccale, cellules somatiques, cellules germinales, et cellules tumorales ou dans lequel les cellules sont des cellules cultivées.

9. Procédé selon l'une quelconque des revendications 1 à 8, dans lequel l'échantillon cellulaire est au moins l'un choisi dans le groupe constitué de sang, salive, muqueuse buccale, ongles, et cheveux, et/ou dans lequel l'échantillon cellulaire est une culture des cellules, et/ou l'échantillon cellulaire est un échantillon cellulaire non dilué.

**10.** Procédé selon l'une quelconque des revendications 1 à 9, dans lequel les cellules sont des cellules procaryotes, et l'étape (A) est une étape de congélation-décongélation ou de réfrigération d'un échantillon cellulaire contenant les cellules procaryotes.

**11.** Procédé selon l'une quelconque des revendications 1 à 10, dans lequel le support est un corps poreux ayant une taille moyenne de pore de 10 µm à 1000 µm, ou dans lequel le support est un tissu non tissé ayant un poids de base de 20 g/m$^3$ à 150 g/m$^3$, ou dans lequel le support est un tissu tissé ayant une taille de maille de 10 µm à 1000 µm.

**12.** Procédé selon l'une quelconque des revendications 1 à 11, dans lequel la forme du support est au moins l'une choisie dans le groupe constitué d'un filtre, une feuille, un bloc, et une bille.

**13.** Procédé selon la revendication 12 dans lequel le support est une bille ayant une fraction volumique de remplissage de 0,1 % à 10 %.

**14.** Procédé selon l'une quelconque des revendications 1 à 13, dans lequel le support est au moins l'un d'un support polymère et d'un support métallique.

**15.** Procédé selon la revendication 14 dans lequel le polymère est au moins l'un choisi dans le groupe constitué de polypropylène, polyéthylène, polyester, polyamide, polyuréthane, polyéther, polystyrène, polychlorure de vinyle, et polytétrafluoroéthylène, et/ou dans lequel le métal est au moins l'un choisi dans le groupe constitué d'acier inoxydable, titane, et aluminium.

**16.** Procédé selon l'une quelconque des revendications 1 à 15, dans lequel l'acide nucléique est au moins l'un de l'ADN et de l'ARN.

**17.** Procédé selon la revendication 16 dans lequel l'ADN est au moins l'un de l'ADN génomique et de l'ADN mitochondrial.

**18.** Procédé de production d'un produit d'amplification d'acide nucléique ayant une séquence cible par un procédé d'amplification d'acide nucléique, comprenant les étapes :

(a) de récupération d'un échantillon d'acide nucléique à partir d'un échantillon cellulaire contenant des cellules par le procédé de production d'un échantillon d'acide nucléique selon l'une quelconque des revendications 1 à 17 ; et
(b) de production, en utilisant de l'acide nucléique dans l'échantillon d'acide nucléique récupéré dans l'étape (a) comme une matrice, d'un produit d'amplification d'acide nucléique ayant une séquence cible dans la matrice par un procédé d'amplification d'acide nucléique.

FIG. 1

FIG. 2A    FIG. 2B    FIG. 2C    FIG. 2D

EP 2 383 335 B1

2

21
22

FIG. 3A

27      24

26      21

A                    A

10    23        25

FIG. 3B

27      24

22

26

10    23        25

FIG. 3C

21
22

26    10    23        25

FIG. 3D

3

21
22

FIG. 4A

27    24    25    31

A                                    A                21

26

10    23                    33

FIG. 4B

27    24    25    31

22

26

10    23                    33

FIG. 4C

21
22

26    10    23            25 33 31

FIG. 4D

## FIG. 5A

## FIG. 5B

## FIG. 5C

FIG. 6A

FIG. 6B

FIG. 6C

FIG. 6D

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2680462 B
- JP 3787354 B
- WO 03006650 A
- JP 9008091 A